(19) Europäisches Patentamt
European Patent Office
Office européen des brevets

(11) Veröffentlichungsnummer: **0 270 061**
**A2**

(12) # EUROPÄISCHE PATENTANMELDUNG

(21) Anmeldenummer: 87117698.8

(22) Anmeldetag: 30.11.87

(51) Int. Cl.4: C07D 271/10 , C07D 413/04 , C07D 249/12 , C07D 285/12 , A01N 43/653 , A01N 43/82

(30) Priorität: 01.12.86 CH 4785/86
16.09.87 CH 3571/87

(43) Veröffentlichungstag der Anmeldung:
08.06.88 Patentblatt 88/23

(84) Benannte Vertragsstaaten:
AT BE CH DE ES FR GB IT LI NL

(71) Anmelder: F. HOFFMANN-LA ROCHE & CO. Aktiengesellschaft

CH-4002 Basel(CH)

(72) Erfinder: Lüthy, Christoph, Dr.
Gfennstrasse 43
CH-8603 Schwerzenbach(CH)

(74) Vertreter: Lederer, Franz, Dr. et al
Patentanwält Dr. Franz Lederer Lucile
Grahnstrasse 22
D-8000 München 80(DE)

(54) **Fünfgliedrige Heterozyklen mit drei Heteroatomen, deren Herstellung und deren Verwendung als Schädlichsbekämpfungsmittel.**

(57) Die Erfindung betrifft neue Verbindungen der Formel

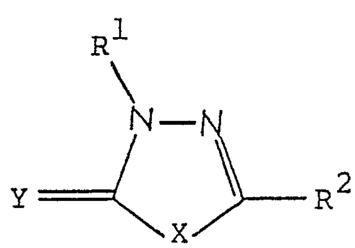

I

worin $R^1$ gegebenenfalls substituiertes Phenyl oder Pyridyl, $R^2$ substituiertes Phenyl, X Sauerstoff, Schwefel oder $NR^3$, Y Sauerstoff oder Schwefel und $R^3$ Methyl, Halogenmethyl oder 2-Propinyl bedeuten, und ihre Herstellung, Schädlingsbekämpfungsmittel, die solche Verbindungen als Wirkstoffe enthalten und die Verwendung der Wirkstoffe bzw. Mittel zur Schädlingsbekämpfung.

EP 0 270 061 A2

F. HOFFMANN-LA ROCHE & CO. Aktiengesellschaft, Basel/Schweiz

30. Nov. 1987

RAN 6101/117

# Fünfgliedrige Heterozyklen mit drei Heteroatomen, deren Herstellung und deren Verwendung als Schädlingsbekämpfungsmittel

Die vorliegende Erfindung betrifft heterocyclische Verbindungen, und zwar 1,3,4-Oxadiazol-2(3H)-one und -thione, 1,3,4-Thiadiazol-2(3H)-one und -thione, und 2,4-Dihydro-3H-1,2,4-triazol-3-one und -thione der allgemeinen Formel

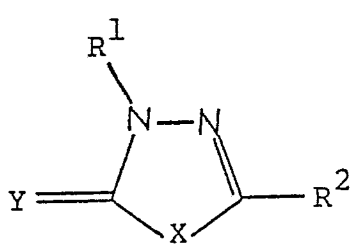

I

worin

$R^1$ gegebenenfalls mit bis zu 4 Halogenatomen, 1 bis 3 $C_{1-4}$-Alkylgruppen, 1 oder 2 Halogenmethylgruppen, einer $C_{1-3}$-Alkoxygruppe, einer $C_{1-2}$-Alkylthiogruppe, einer $C_{1-3}$-Halogenalkoxygruppe, einer $C_{1-2}$-Halogenalkylthiogruppe und/oder einer Cyanogruppe substituiertes Phenyl oder gegebenenfalls mit 1 oder 2 Halogenatomen, 1 oder 2 Methylgruppen oder einer Halogenmethylgruppe substituiertes Pyridyl,

Pa/15.9.87

$R^2$ mit bis zu 3 Halogenatomen, einer Methylgruppe, einer Halogenmethylgruppe und/oder 1 oder 2 Methoxygruppen substituiertes Phenyl, wobei mindestens eine der beiden o-Stellungen besetzt ist,

X Sauerstoff, Schwefel oder $NR^3$,

Y Sauerstoff oder Schwefel, und

$R^3$ Methyl, Halogenmethyl oder 2-Propinyl bedeuten.

Die Verbindungen der Formel I sind Schädlingsbekämpfungsmittel und eignen sich insbesondere zur Bekämpfung von Insekten und Milben, z.B. Spinnmilben. Somit umfasst die Erfindung auch Schädlingsbekämpfungsmittel, welche Verbindungen der Formel I als Wirkstoffe enthalten, ein Verfahren zur Herstellung dieser Verbindungen sowie die Verwendung dieser Verbindungen bzw. Mittel zur Bekämpfung von Schädlingen.

Der in obiger Definition der Verbindungen der Formel I verwendete Ausdruck "Halogen" umfasst Fluor, Chlor, Brom und Jod. Die Gruppen "Halogenmethyl", "$C_{1-3}$-Halogenalkoxy" und "$C_{1-2}$-Halogenalkylthio" können jeweils einen oder mehrere (gleiche oder verschiedene) Halogensubstituenten aufweisen. Auch in der substituierten Phenylgruppe $R^1$ bzw. $R^2$ sowie im Falle von $R^1$ = substituiertes Pyridyl können die Substituenten gleich oder verschieden sein. Diejenigen Alkyl-, Alkoxy- bzw. Halogenalkoxygruppen, die 3 oder 4 Kohlenstoffatome enthalten, können geradkettig oder verzweigt sein.

Bedeutet $R^1$ mit bis zu 4 Halogenatomen substituiertes Phenyl, weist dieses insbesondere 1 bis 4 Fluoratome, 1 bis 3 Chloratome, ein Bromatom und/oder ein Jodatom auf. Bedeutet $R^1$ hingegen mit 1 oder 2 Halogenatomen substituiertes Pyridyl, weist dieses insbesondere 1 oder 2 Fluoratome, 1 oder 2 Chloratome oder ein Bromatom auf. Im Falle von $R^2$, das mit bis zu 3 Halogenatomen substituiertes Phenyl bedeuten kann, sind als Substituenten insbesondere 1

bis 3 Fluoratome, 1 oder 2 Chloratome, ein Bromatom und/oder ein Jodatom vorhanden.

$R^1$ bedeutet vorzugsweise eine mono- oder disubstituierte Phenylgruppe, in der die Substituenten ein oder zwei Fluoratome, ein oder zwei Chloratome, ein Bromatom, eine oder zwei Methylgruppen, eine Trifluormethylgruppe und/oder eine Halogenmethoxygruppe sind und eine o-Stellung besetzt ist. Besonders bevorzugt sind in diesem Fall Fluor, Chlor, Brom, Methyl oder Trifluormethyl.

Unabhängig von der Bedeutung von $R^1$ bedeutet $R^2$ vorzugsweise eine mono- oder disubstituierte Phenylgruppe, in der die Substituenten ein oder zwei Fluoratome, ein oder zwei Chloratome und/oder ein Bromatom sind. Besonders bevorzugt bedeutet $R^2$ 2-Chlorphenyl, 2-Chlor-4-fluorphenyl, 2-Chlor-6-fluorphenyl oder 2,6-Difluorphenyl.

X steht vorzugsweise für Sauerstoff.

Bevorzugte einzelne Verbindungen der Formel I sind:

5-(2-Chlor-6-fluorphenyl)-3-(α,α,α-trifluor-o-tolyl)-1,3,4-oxadiazol-2(3H)-on,
5-(2-Chlor-6-fluorphenyl)-3-(2-chlorphenyl)-1,3,4-oxadiazol-2(3H)-on,
5-(2,6-Difluorphenyl)-3-(α,α,α-trifluor-o-tolyl)-1,3,4-oxadiazol-2(3H)-on,
5-(2-Chlor-4-fluorphenyl)-3-(2-chlorphenyl)-1,3,4-oxadiazol-2(3H)-on,
5-(2-Chlor-6-fluorphenyl)-3-(3-chlor-o-tolyl)-1,3,4-oxadiazol-2(3H)-on,
5-(2-Chlor-6-fluorphenyl)-3-(2-chlorphenyl)-1,3,4-oxadiazol-2(3H)-thion,
3-(2-Chlorphenyl)-5-(2,6-difluorphenyl)-1,3,4-oxadiazol-2(3H)-on,

3-(2-Chlorphenyl)-5-(2,6-dichlorphenyl)-1,3,4-oxadiazol-2(3H)-on,

5-(2-Chlorphenyl)-3-(α,α,α-trifluor-o-tolyl)-1,3,4-oxadiazol-2(3H)-on,

5-(2,6-Difluorphenyl)-3-(α,α,α-trifluor-o-tolyl)-1,3,4-oxadiazol-2(3H)-thion,

3-(2-Bromphenyl)-5-(2,6-difluorphenyl)-1,3,4-oxadiazol-2(3H)-on und

5-(2-Chlor-6-fluorphenyl)-3-(2-fluorphenyl)-1,3,4-oxadiazol-2(3H)-on.

Weitere Vertreter von Verbindungen der Formel I sind:

5-(2-Chlorphenyl)-3-(2-fluorphenyl)-1,3,4-oxadiazol-2(3H)-on,

3-(2,4-Dichlorphenyl)-5-(2,6-difluorphenyl)-1,3,4-oxadiazol-2(3H)-on,

3-(2-Chlor-4-fluorphenyl)-5-(2-chlor-6-fluorphenyl)-1,3,4-oxadiazol-2(3H)-on,

5-(2,4-Difluorphenyl)-3-(α,α,α-trifluor-o-tolyl)-1,3,4-oxadiazol-2(3H)-on,

5-(2-Chlor-6-fluorphenyl)-3-(2-chlor-4-trifluormethyl-phenyl)-1,3,4-oxadiazol-2(3H)-on,

3-(3,5-Dichlor-2,4-difluorphenyl)-5-(2,6-difluorphenyl)-1,3,4-oxadiazol-2(3H)-on,

3-(2-Chlorphenyl)-5-(2-fluorphenyl)-1,3,4-oxadiazol-2(3H)-on,

5-(2-Bromphenyl)-3-(2-chlorphenyl)-1,3,4-oxadiazol-2(3H)-on,

3-(3-Chlor-o-tolyl)-5-(2,6-difluorphenyl)-1,3,4-oxadiazol-2(3H)-on,

3-(2,5-Dichlor-3-trifluormethyl-phenyl)-5-(2,6-difluorphenyl)-1,3,4-oxadiazol-2(3H)-on,

5-(2-Chlor-6-fluorphenyl)-3-(2-trifluormethoxy-phenyl)-1,3,4-oxadiazol-2(3H)-on,

5-(2-Chlor-4-fluorphenyl)-3-(2-methoxy-m-tolyl)-1,3,4-oxadiazol-2(3H)-on,

3-(2-Aethoxy-m-tolyl)-5-(2-chlorphenyl)-1,3,4-oxadiazol-2(3H)-on,

3-(2-Aethoxy-3-äthylphenyl)-5-(2,6-difluorphenyl)-1,3,4-oxadiazol-2(3H)-on,

5-(2-Chlorphenyl)-3-(2-isopropoxy-m-tolyl)-1,3,4-oxadiazol-2(3H)-on,

5-(2-Chlor-6-fluorphenyl)-3-(2-methoxy-p-tolyl)-1,3,4-oxadiazol-2(3H)-on,

5-(2-Chlorphenyl)-3-(4-fluor-2-methoxyphenyl)-1,3,4-oxadiazol-2(3H)-on,

5-(2-Chlorphenyl)-3-(4-trifluormethoxy-phenyl)-1,3,4-oxadiazol-2(3H)-on,

3-[3,5-Dichlor-4-(1,1,2,2-tetrafluoräthoxy)-phenyl]-5-(2,6-difluorphenyl)-1,3,4-oxadiazol-2(3H)-on,

5-(2-Chlorphenyl)-3-[2,5-dichlor-4-(1,1,2,3,3,3-hexafluorpropoxy)-phenyl]-1,3,4-oxadiazol-2(3H)-on,

5-(2-Chlor-6-fluorphenyl)-3-(2-chlor-5-trifluormethylphenyl)-1,3,4-oxadiazol-2(3H)-on,

5-(2,6-Difluorphenyl)-3-(α,α,α-trifluor-p-tolyl)-1,3,4-oxadiazol-2(3H)-on,

3-(2-Chlorphenyl)-5-(2-chlor-4,6-difluorphenyl)-1,3,4-oxadiazol-2(3H)-on,

5-(2-Chlor-6-fluorphenyl)-3-(o-tolyl)-1,3,4-oxadiazol-2(3H)-on,

3-(3-Chlor-2-trifluormethyl-phenyl)-5-(2,6-difluorphenyl)-1,3,4-oxadiazol-2(3H)-on,

3-(2-Cyanophenyl)-5-(2,6-difluorphenyl)-1,3,4-oxadiazol-2(3H)-on,

3-(2-Chlorphenyl)-5-(2-chlor-5-fluorphenyl)-1,3,4-oxadiazol-2(3H)-on,

5-(3-Chlor-2,4-difluorphenyl)-5-(2-chlorphenyl)-1,3,4-oxadiazol-2(3H)-on,

3-(3-Chlor-4-trifluormethyl-phenyl)-5-(2,6-difluorphenyl)-1,3,4-oxadiazol-2(3H)-on,

3-(3,5-Dichlor-o-tolyl)-5-(2,6-difluorphenyl)-1,3,4-oxadiazol-2(3H)-on,

3-(2,3-Dichlorphenyl)-5-(2,6-difluorphenyl)-1,3,4-oxadiazol-2(3H)-on,

3-(3-Chlor-2-pyridyl)-5-(2,6-difluorphenyl)-1,3,4-oxadiazol-2(3H)-on,

5-(2-Chlor-6-fluorphenyl)-3-(α,α,α-trifluor-o-tolyl)-1,3,4-oxadiazol-2(3H)-thion,

5-(2-Chlor-4-fluorphenyl)-3-(α,α,α-trifluor-o-tolyl)-1,3,4-oxadiazol-2(3H)-thion,

5-(2,6-Difluorphenyl)-3-(2-methoxy-m-tolyl)-1,3,4-oxadiazol-2(3H)-thion,

5-(2-Chlor-6-fluorphenyl)-3-(3-chlor-o-tolyl)-1,3,4-oxadiazol-2(3H)-thion,

5-(2-Chlor-6-fluorphenyl)-2-(2,3-dichlorphenyl)-4-methyl-2,4-dihydro-3H-1,2,4-triazol-3-on,

5-(2-Chlorphenyl)-4-methyl-2-(2,6-dichlor-4-pyridyl)-2,4-dihydro-3H-1,2,4-triazol-3-on,

4-Difluormethyl-5-(2,6-difluorphenyl)-2-(α,α,α-trifluor-o-tolyl)-2,4-dihydro-3H-1,2,4-triazol-3-on,

2-(2-Chlorphenyl)-5-(2-chlor-6-fluorphenyl)-4-difluormethyl-2,4-dihydro-3H-1,2,4-triazol-3-on,

2-(2-Chlorphenyl)-4-difluormethyl-5-(2,6-difluorphenyl)-2,4-dihydro-3H-1,2,4-triazol-3-on,

5-(2-Chlor-4-fluorphenyl)-4-difluormethyl-2-(α,α,α-trifluor-o-tolyl)-2,4-dihydro-3H-1,2,4-triazol-3-on,

2-(3-Chlor-o-tolyl)-5-(2,6-difluorphenyl)-4-methyl-2,4-dihydro-3H-1,2,4-triazol-3-on,

2-(2-Bromphenyl)-5-(2,6-difluorphenyl)-4-methyl-2,4-dihydro-3H-1,2,4-triazol-3-on,

2-(2-Bromphenyl)-5-(2-chlor-6-fluorphenyl)-4-methyl-2,4-dihydro-3H-1,2,4-triazol-3-on,

5-(2-Chlor-4-fluorphenyl)-4-methyl-2-(α,α,α-trifluor-o-tolyl)-2,4-dihydro-3H-1,2,4-triazol-3-on,

5-(2,6-Difluorphenyl)-4-methyl-2-(α,α,α-trifluor-o-tolyl)-2,4-dihydro-3H-1,2,4-triazol-3-thion,

3-(2-Chlorphenyl)-5-(2,4-dichlorphenyl)-1,3,4-oxadiazol-2(3H)-on,

5-(2-Chlor-4-fluorphenyl)-3-(2,6-difluorphenyl)-1,3,4-oxadiazol-2(3H)-on,

3-(2-Chlor-5-trifluormethyl-phenyl)-5-(2,6-difluorphenyl)-1,3,4-oxadiazol-2(3H)-on,

2-(3,4-Dichlorphenyl)-5-(2,6-difluorphenyl)-4-methyl-2,4-dihydro-3H-1,2,4-triazol-3-on,

5-(2,6-Difluorphenyl)-3-(2-trifluormethoxy-phenyl)-1,3,4-oxadiazol-2(3H)-on,

5-(2-Chlor-6-fluorphenyl)-3-(2-difluormethoxy-phenyl)-1,3,4-oxadiazol-2(3H)-on,

3-[2,5-Dichlor-4-(1,1,2,2-tetrafluoräthoxy)-phenyl]-5-(2,6-difluorphenyl)-1,3,4-oxadiazol-2(3H)-on,

5-(2,6-Difluorphenyl)-3-[4-(1,1,2,2-tetrafluoräthoxy)-3-trifluormethyl-phenyl)-1,3,4-oxadiazol-2(3H)-on,

5-(2-Chlor-6-fluorphenyl)-3-(4-trifluormethoxy-3-trifluormethyl-phenyl)-1,3,4-oxadiazol-2(3H)-on,

3-[4-(2-Chlor-1,1,2-trifluoräthoxy)-3,5-dichlorphenyl]-5-(2,6-difluorphenyl)-1,3,4-oxadiazol-2(3H)-on,

3-[3,5-Dichlor-4-(1,1,2,3,3,3-hexafluorpropoxy)-phenyl]-5-(2,6-difluorphenyl)-1,3,4-oxadiazol-2(3H)-on,

5-(2-Chlor-6-fluorphenyl)-3-(2-chlor-4-trifluormethylthio-phenyl)-1,3,4-oxadiazol-2(3H)-on,

5-(2-Methoxyphenyl)-3-(α,α,α-trifluor-o-tolyl)-1,3,4-oxadiazol-2(3H)-on,

3-(4-Chlor-3,5-difluorphenyl)-5-(2-chlor-6-fluorphenyl)-1,3,4-oxadiazol-2(3H)-on,

3-(2,4-Difluorphenyl)-5-(2,6-difluorphenyl)-1,3,4-oxadiazol-2(3H)-on,

5-(2,6-Difluorphenyl)-3-(2,3,4-trichlorphenyl)-1,3,4-oxadiazol-2(3H)-on,

3-(4-Brom-2-fluor-5-trifluormethyl-phenyl)-5-(2,6-difluorphenyl)-1,3,4-oxadiazol-2(3H)-on,

5-(2,6-Difluorphenyl)-3-(4-fluor-3-trifluormethyl-phenyl)-1,3,4-oxadiazol-2(3H)-on,

3-(2-Brom-4-trifluormethyl-phenyl)-5-(2,6-difluorphenyl)-1,3,4-oxadiazol-2(3H)-on,

5-(2-Chlor-6-fluorphenyl)-3-(2-fluor-5-trifluormethyl-phenyl)-1,3,4-oxadiazol-2(3H)-on,

5-(2-Chlorphenyl)-3-(2,6-difluor-4-trifluormethyl-phenyl)-1,3,4-oxadiazol-2(3H)-on,

5-(2,4,6-Trifluorphenyl)-3-(α,α,α-trifluor-o-tolyl)-1,3,4-oxadiazol-2(3H)-on,

5-(2-Chlor-6-fluorphenyl)-3-(2-difluormethyl-phenyl)-1,3,4-oxadiazol-2(3H)-on,

5-(2-Chlor-4-fluorphenyl)-3-(5-chlor-2,3,4-trifluorphenyl)-1,3,4-oxadiazol-2(3H)-on,

3-(3-Chlor-2-fluor-4-trifluormethoxy-phenyl)-5-(2,6-difluorphenyl)-1,3,4-oxadiazol-2(3H)-on,

3-(2,3-Dichlor-5-trifluormethyl-phenyl)-5-(2,6-difluorphenyl)-1,3,4-oxadiazol-2(3H)-on,

3-(3-Chlor-2-fluor-5-trifluormethyl-phenyl)-5-(2,6-difluorphenyl)-1,3,4-oxadiazol-2(3H)-on,

3-(2-Chlor-3-trifluormethyl-phenyl)-5-(2,6-difluorphenyl)-1,3,4-oxadiazol-2(3H)-on,

5-(2-Chlor-6-fluorphenyl)-3-(3-fluor-o-tolyl)-1,3,4-oxadiazol-2(3H)-on,

3-(3-Chlor-2,5-dimethylphenyl)-5-(2-chlor-6-fluorphenyl)-1,3,4-oxadiazol-2(3H)-on,

5-(2-Chlor-6-fluorphenyl)-3-(2,3,5-trifluorphenyl)-1,3,4-oxadiazol-2(3H)-on,

5-(2,6-Difluorphenyl)-3-[2-(1,1,2,2-tetrafluoräthoxy)-phenyl]-1,3,4-oxadiazol-2(3H)-on,

3-(4-Chlor-3-chlordifluormethoxy-phenyl)-5-(2,6-difluorphenyl)-1,3,4-oxadiazol-2(3H)-on,

3-(2-Chlorphenyl)-5-(2-fluor-6-methoxyphenyl)-1,3,4-oxadiazol-2(3H)-on,

3-(2-Chlorphenyl)-5-(2-trifluormethoxy-phenyl)-1,3,4-oxadiazol-2(3H)-on,

5-(2-Chlor-6-fluorphenyl)-3-[2-(2-chloräthoxy)-phenyl]-1,3,4-oxadiazol-2(3H)-on,

3-(3-Chlor-2-fluorphenyl)-5-(2-chlor-6-fluorphenyl)-1,3,4-oxadiazol-2(3H)-on,

3-(3-Chlor-2-fluorphenyl)-5-(2-chlor-6-fluorphenyl)-1,3,4-oxadiazol-2(3H)-thion,

5-(2-Chlor-6-fluorphenyl)-3-(2,3-dichlorphenyl)-1,3,4-oxadiazol-2(3H)-on,

5-(2-Chlorphenyl)-3-(3-chlor-o-tolyl)-1,3,4-oxadiazol-2(3H)-on,

3-(3-Chlor-2-fluorphenyl)-5-(2,6-difluorphenyl)-1,3,4-oxadiazol-2(3H)-on,

3-(2-Chlor-6-fluor-4-trifluormethyl-phenyl)-5-(2,6-difluorphenyl)-1,3,4-oxadiazol-2(3H)-on,

3-(2-Chlor-4-trifluormethoxy-phenyl)-5-(2,6-difluorphenyl)-1,3,4-oxadiazol-2(3H)-on,

5-(2-Chlor-6-fluorphenyl)-3-(2-fluor-4-trifluormethylthio-phenyl)-1,3,4-oxadiazol-2(3H)-on,

3-[4-(2-Chlor-1,1,2-trifluoräthylthio)-2-fluorphenyl]-5-(2,6-difluorphenyl)-1,3,4-oxadiazol-2(3H)-on,

5-(2,6-Difluorphenyl)-3-(4-fluor-2-trifluormethoxy-phenyl)-1,3,4-oxadiazol-2(3H)-on,

5-(2-Chlorphenyl)-3-[4-(2,2-dichlor-1,1-difluoräthoxy)-phenyl]-1,3,4-oxadiazol-2(3H)-on,

5-(2,6-Difluorphenyl)-3-(4-trifluormethoxy-3-trifluormethyl-phenyl)-1,3,4-oxadiazol-2(3H)-on,

3-(2-Chlor-m-tolyl)-5-(2,6-difluorphenyl)-1,3,4-oxadiazol-2(3H)-thion,

5-(2-Chlor-6-fluorphenyl)-3-(2,3-dichlorphenyl)-1,3,4-oxadiazol-2(3H)-thion,

3-(2-Chlorphenyl)-5-(2-chlor-4-fluorphenyl)-1,3,4-oxadiazol-2(3H)-thion,

3-(2-Bromphenyl)-5-(2,6-difluorphenyl)-1,3,4-oxadiazol-2(3H)-thion,

3-(2-Chlorphenyl)-5-(2,6-difluorphenyl)-1,3,4-oxadiazol-2(3H)-thion,

5-(2-Chlor-6-fluorphenyl)-3-(2-fluorphenyl)-1,3,4-oxadiazol-2(3H)-thion,

5-(2-Chlorphenyl)-3-(α,α,α-trifluor-o-tolyl)-1,3,4-oxadiazol-2(3H)-thion,

5-(2-Chlorphenyl)-3-(3-chlor-o-tolyl)-1,3,4-oxadiazol-2(3H)-thion,

3-(3-Chlor-2-fluorphenyl)-5-(2,6-difluorphenyl)-1,3,4-oxadiazol-2(3H)-thion,

3-(2,3,4,5-Tetrafluorphenyl)-5-(2,4,6-trifluorphenyl)-1,3,4-oxadiazol-2(3H)-thion,

5-(2,6-Difluorphenyl)-3-(2-fluorphenyl)-1,3,4-oxadiazol-2(3H)-on,

5-(2-Chlor-6-fluorphenyl)-3-(2-chlor-3-pyridyl)-1,3,4-oxadiazol-2(3H)-on,

3-(2-Chlor-5-methoxy-phenyl)-5-(α,α,α-trifluor-o-tolyl)-1,3,4-oxadiazol-2(3H)-on,

3-(3,4-Dichlorphenyl)-5-(α,α,α-trifluor-o-tolyl)-1,3,4-oxadiazol-2(3H)-on,

5-(2,6-Difluorphenyl)-3-(α,α,α-trifluor-o-tolyl)-1,3,4-thiadiazol-2(3H)-thion,

3-(2,6-Dichlor-4-trifluormethoxy-phenyl)-5-(2,6-difluorphenyl)-1,3,4-oxadiazol-2(3H)-on,

3-[2-Chlor-4-(1,1,2,3,3,3-hexafluorpropoxy)-3-trifluormethyl-phenyl]-5-(2,6-difluorphenyl)-1,3,4-oxadiazol-2(3H)-on,

5-(2-Chlor-6-fluorphenyl)-3-(2,4-dimethylphenyl)-1,3,4-oxadiazol-2(3H)-thion,

3-(3-Chlor-o-tolyl)-5-(2,6-difluorphenyl)-1,3,4-oxadiazol-2(3H)-thion,

3-(2,6-Dichlor-4-trifluormethyl-phenyl)-5-(2,6-difluorphenyl)-1,3,4-oxadiazol-2(3H)-thion,

3-(2,6-Dichlor-4-trifluormethoxy-phenyl)-5-(2,6-difluorphenyl)-1,3,4-oxadiazol-2(3H)-thion und

3-[2-Chlor-4-(2-chlor-1,1,2-trifluoräthoxy)-5-trifluormethyl-phenyl]-5-(2,6-difluorphenyl)-1,3,4-oxadiazol-2(3H)-thion.

Das erfindungsgemässe Verfahren zur Herstellung der Verbindungen der Formel I ist dadurch gekennzeichnet, dass man

a) zwecks Herstellung derjenigen Verbindungen der Formel

I, in denen X Sauerstoff oder Schwefel bedeutet, ein Hydrazid der allgemeinen Formel

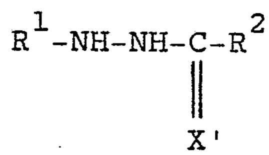

II

worin $R^1$ und $R^2$ die oben angegebenen Bedeutungen besitzen und X' Sauerstoff oder Schwefel bedeutet, mit Phosgen, Chlorameisensäure-trichlormethylester oder einem Chlorameisensäure-niederalkylester (Y= Sauerstoff) bzw. mit Thiophosgen oder Schwefelkohlenstoff (Y= Schwefel) umsetzt,

b) zwecks Herstellung derjenigen Verbindungen der Formel I, in denen X $NR^3$ und Y Sauerstoff bedeuten, ein 2,4-Dihydro-3H-1,2,4-triazol-3-on der allgemeinen Formel

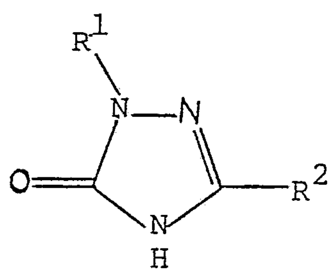

III

worin $R^1$ und $R^2$ die oben angegebenen Bedeutungen besitzen,

einer Alkylierung unterwirft, oder

c) zwecks Herstellung derjenigen Verbindungen der Formel I, in denen Y Schwefel bedeutet, eine Verbindung der allgemeinen Formel

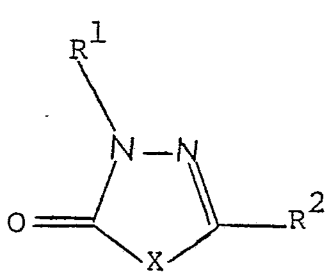

I'

worin $R^1$, $R^2$ und X die oben angegebenen Bedeutungen besitzen,

mit einem Schwefelungsmittel behandelt.

Die Umsetzung nach Verfahrensvariante a) wird im Falle der Verwendung von Phosgen, Chlorameisensäure-trichlormethylester oder Thiophosgen zweckmässigerweise so durchgeführt, dass man eine Lösung oder Suspension des Hydrazids der Formel II in einem inerten Lösungs- bzw. Verdünnungsmittel, wie einem chlorierten aliphatischen Kohlenwasserstoff, z.B. Methylenchlorid oder Chloroform; einem aromatischen Kohlenwasserstoff, z.B. Toluol; einem Essigsäure--niederalkylester, z.B. Aethylacetat; oder einem aliphatischen oder cyclischen Aether, z.B. Dioxan, mit einer Lösung von Phosgen bzw. Thiophosgen in demselben Lösungs- bzw. Verdünnungsmittel, insbesondere in Toluol; oder mit flüssigem Chlorameisensäure-trichlormethylester ("Diphosgen") behandelt, und zwar vorzugsweise bei Temperaturen zwischen der Raumtemperatur und der Rückflusstemperatur des Reaktionsgemisches, besonders bevorzugt im Temperaturbereich von 55°C bis 110°C. Dabei wird vorzugsweise mit einem geringen Ueberschuss an Phosgen, Diphosgen oder Thiophosgen gearbeitet, wobei das Molverhältnis Hydrazid II: Phosgen oder Thiophosgen bzw. Diphosgen ca. 1:1,05-5 bzw. 1:0,55-2,5 beträgt.

Es kann bei der Umsetzung vorerst zumindest teilweise eine Additionsreaktion stattfinden, wobei ein Zwischenprodukt der allgemeinen Formel IV

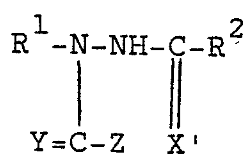

IV

worin $R^1$, $R^2$, X' und Y die oben angegebenen Bedeutungen besitzen und Z Chlor oder Trichlormethoxy bedeutet,

gebildet wird. Die Cyclisierung von IV kann dadurch erfolgen, dass man das überschüssige Phosgen bzw. Thiosphosgen abdampft, das verbleibende Rohgemisch erneut in ein inertes Lösungsmittel, wie einen chlorierten aliphatischen Kohlenwasserstoff, z.B. Methylenchlorid; einen aromatischen Kohlenwasserstoff, z.B. Toluol; ein niederes Alkanol, z.B. Methanol; oder einen aliphatischen oder cyclischen Aether, z.B. Tetrahydrofuran, aufnimmt, und die Lösung in Gegenwart einer Hilfsbase, z.B. von Triäthylamin, gegebenenfalls mit Alkyl substituiertem Pyridin, 4-Dimethylaminopyridin oder N,N-Dimethylanilin, kurze Zeit auf Rückflusstemperatur erhitzt. Zweckmässigerweise beträgt das Molverhältnis eingesetzte Base:Hydrazid II ca. 1,0 bis 2,0:1.

Im Falle der Verwendung eines Chlorameisensäure-niederalkylesters, insbesondere des Aethylesters, als zweiten Reaktionsteilnehmers erfolgt die Umsetzung zweckmässigerweise in Gegenwart eines Verdünnungsmittels, wie eines chlorierten aliphatischen Kohlenwasserstoffes, z.B. Chloroform; eines aromatischen Kohlenwasserstoffes, z.B. Toluol; eines aliphatischen oder cyclischen Aethers, z.B. Tetrahydrofuran; oder Acetonitril, sowie unter Verwendung einer Hilfsbase, z.B. Triäthylamin, bei Temperaturen von 0°C bis zur Rückflusstemperatur des Reaktionsgemisches. Es werden zweckmässigerweise stöchiometrische Mengen Hydrazid und Ester eingesetzt, oder es wird mit einem geringen Ueberschuss, und zwar bis zu einem 40%-igen Ueberschuss, am Ester gearbeitet. Auch die Hilfsbase wird in stöchiometrischer Menge oder in einem geringen Ueberschuss, also bis zu ca. 40%-igem Ueberschuss, eingesetzt. Bei dieser Umsetzung findet vorerst eine Additionsreaktion statt, wobei ein Zwischenprodukt der oben angegebenen allgemeinen Formel IV, in der $R^1$, $R^2$, X' und Y die oben angegebenen Bedeutungen besitzen und Z Niederalkoxy, insbesondere Aethoxy, bedeutet, gebildet wird. Dieses Zwischenprodukt wird dann durch Erhitzen cyclisiert, und zwar gegebenenfalls in Gegenwart eines Verdünnungsmittels, wie eines gegebenenfalls chlorier-

ten aromatischen Kohlenwasserstoffes, z.B. Xylol oder o-Dichlorbenzol, und zweckmässigerweise unter Verwendung einer katalytischen Menge einer Base, z.B. Dimethylaminopyridin. Besonders bevorzugt wird jedoch in der Schmelze, also im Temperaturbereich von 120°C bis 200°C, cyclisiert. Für weitere Details über die Umsetzung eines Hydrazides mit einem Chlorameisensäure-niederalkylester wird auf J. Het. Chem., 23, 417 (1986) verwiesen.

Im Falle der Verwendung von Schwefelkohlenstoff als zweitem Reaktionsteilnehmer in der Verfahrensvariante a) erfolgt die Umsetzung zweckmässigerweise ebenfalls in Gegenwart eines Verdünnungsmittels, wie beispielsweise Aethanol, Methylcellosolve oder Wasser, sowie unter Verwendung einer Base, z.B. Kaliumhydroxid, Natriummethylat oder Dimethylamin, bei Temperaturen von 0°C bis zur Rückflusstemperatur des Reaktionsgemisches. Dabei wird der Schwefelkohlenstoff und die Base jeweils in stöchiometrischer Menge oder in einem geringen Ueberschuss von bis zu ca. 40%-igem eingesetzt. Auch bei dieser Umsetzung findet vorerst eine Additionsreaktion statt, wobei in diesem Fall ein Zwischenprodukt der oben angegebenen allgemeinen Formel IV, in der $R^1$, $R^2$ und X' die oben angegebenen Bedeutungen besitzen und Y und Z Schwefel bedeuten, als Salz erhalten wird. Dieses Salz wird dann durch Behandlung mit einer Mineralsäure, z.B. Salzsäure, cyclisiert, und zwar zweckmässigerweise in demselben Verdünnungsmittel und/oder Wasser.

Bei der Verfahrensvariante b) steht der Ausdruck "Alkylierung" für die Einführung einer Methyl-, Halogenmethyl- oder 2-Propinylgruppe ($R^3$) am unsubstituierten Stickstoffatom (-NH-) des Triazolkerns. Als Alkylierungsmittel wird zweckmässigerweise ein Methyl-, Halogenmethyl- oder 2-Propinylhalogenid, z.B. Methyljodid, Difluorchlormethan oder Propargylbromid, oder Dimethylsulfat, verwendet. Die Alkylierung erfolgt zweckmässigerweise in einem inerten Verdünnungsmittel, in dem das Ausgangsmaterial III entweder

gelöst oder suspendiert ist, und zudem vorzugsweise in Gegenwart eines säurebindenden Mittels. Als Verdünnungsmittel eignen sich insbesondere aliphatische Ketone, z.B. Aceton und Methyläthylketon; aliphatische und cyclische Aether, z.B. Dimethoxyäthan und Tetrahydrofuran, aromatische Kohlenwasserstoffe, z.B. Toluol; Acetonitril; Dimethylformamid; Dimethylpropylenharnstoff; Dimethylsulfoxid; Alkohole, z.B. Aethanol und Methylcellosolve; sowie Wasser. Beispiele geeigneter säurebindender Mittel sind Natriumhydrid; Alkalimetallcarbonate, z.B. Natrium- und Kaliumcarbonat; Alkalimetallhydrogencarbonate, z.B. Natriumhydrogencarbonat; und Trialkylamine, z.B. Triäthylamin. Diese Mittel werden vorzugsweise in stöchiometrischer Menge oder sogar in Ueberschuss, d.h. im Moläquivalentbereich von 1,0 bis ca. 5,0, verwendet. Zudem sind vorzugsweise katalytische Mengen (ca. 1 bis 10 Gewichtsprozent) Dimethylaminopyridin vorhanden. Die Reaktionstemperaturen können bei der Durchführung dieser Verfahrensvariante in einem grossen Bereich variiert werden, wobei im allgemeinen bei Temperaturen zwischen -20°C und 160°C, vorzugsweise zwischen 20°C und 80°C, gearbeitet wird.

Es kann sich bei der Alkylierung ein Nebenprodukt der allgemeinen Formel

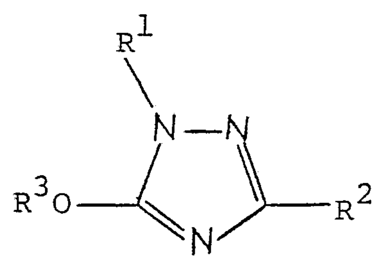

worin $R^1$, $R^2$ und $R^3$ die oben angegebenen Bedeutungen besitzen,

bilden. Dieses Nebenprodukt ist normalerweise in verhältnismässig kleiner Menge, also bis ca. 5 Gewichtsprozent, vorhanden und besitzt selber pestizide Eigenschaften, wenn auch im allgemeinen weniger ausgeprägt als im Falle der erfindungsgemässen Verbindungen I. Das Nebenprodukt kann nach üblichen Methoden, z.B. Chromatographie oder fraktionierte

Kristallisation, vom Hauptprodukt der Formel I abgetrennt werden.

Zur Schwefelung nach Verfahrensvariante c) wird zweckmässigerweise Phosphorpentasulfid, gegebenenfalls in Gegenwart von Pyridin, z.B. als Phosphorpentasulfid-Pyridin (1:2)-Komplex, das Lawesson-Reagens 2,4-Bis-(4-methoxyphenyl)-1,2-dithioxo -1,3,2,4-dithiaphosphetan [siehe z.B. S.-O. Lawesson et al., Bull. Soc. Chim. Belg. 87, 229-238 (1978)] oder das Davy-Reagens 2,4-Bis-(methylthio)-1,3,2,4--dithiadiphosphetan (siehe z.B. Sulfur Lett. 1983, 1, 167) verwendet, wobei dieses vorzugsweise in stöchiometrischer Menge oder in geringem Ueberschuss (z.B. bis zu 20%) eingesetzt wird. Zweckmässigerweise wird in einem inerten organischen Verdünnungsmittel, wie einem gegebenenfalls halogenierten Aromaten, z.B. Toluol oder Dichlorbenzol, oder einem aliphatischen oder cyclischen Aether, z.B. Dimethoxyäthan, und bei erhöhter Temperatur, insbesondere bei Temperaturen zwischen 80°C und der Rückflusstemperatur des Reaktionsgemisches, gearbeitet. Zudem wird vorteilhaft eine katalytische Menge, also ca. 0,1 bis 10 Gewichtsprozent, bezogen auf die Menge der Verbindung I', Hexamethylphosphortriamid zugesetzt.

Die Isolierung und die Reinigung der so hergestellten Verbindungen der Formel I können nach an sich bekannten Methoden, z.B. Umkristallisieren, Destillieren oder Säulenchromatographie, erfolgen.

Die als Ausgangsmaterialien in der Verfahrensvariante a) verwendeten Hydrazide der Formel II, in denen X' Sauerstoff bedeutet, sind entweder bekannt oder können in an sich bekannter Weise hergestellt werden, beispielsweise durch Umsetzung von entsprechenden Hydrazinen der allgemeinen Formel

$$R^1\text{-NH-NH}_2 \qquad \text{V}$$

worin $R^1$ die oben angegebene Bedeutung besitzt, oder deren Säureadditionssalzen, z.B. Hydrochloriden, mit substituierten Benzoesäurehalogeniden oder -niederalkylestern der allgemeinen Formel

$$R^2-COR^4 \qquad VI$$

worin $R^2$ die oben angegebene Bedeutung besitzt und $R^4$ Fluor, Chlor oder Brom, oder nieder Alkoxy, insbesondere $C_{1-3}$-Alkoxy, bedeutet, in Gegenwart einer Base, wie beispielsweise Pyridin, Triäthylamin, Natriumhydroxid, Calciumhydroxid, Kaliumcarbonat oder Calciumoxid, unter den dem Fachmann geläufigen Reaktionsbedingungen. So behandelt man beispielsweise das Hydrazin V oder ein Säureadditionssalz davon in einem inerten Verdünnungsmittel, wie einem aliphatischen oder aromatischen Kohlenwasserstoff, z.B. Toluol, einem aliphatischen oder cyclischen Aether, z.B. tert.Butylmethyläther oder Tetrahydrofuran; einem aliphatischen Ester, z.B. Aethylacetat; einem Alkohol, z.B. Aethanol; Wasser; oder einem Gemisch von zwei oder mehreren dieser Verdünnungsmittel, mit dem Benzoesäurederivat VI. In einer bevorzugten Ausführungsform wird ein Säureadditionssalz, z.B. das Hydrochlorid, des Hydrazins V in Wasser vorgelegt und das wässrige Medium mit einem mit Wasser nichtmischbaren organischen Lösungsmittel, z.B. Toluol, tert.Butylmethyläther oder - vorzugsweise - Aethylacetat, überschichtet. Durch Zusatz einer anorganischen Base als säurebindenden Mittels, z.B. Natriumhydroxid, Natrium- oder Kaliumcarbonat, oder Calciumoxid, wird vorerst das Hydrazin V freigesetzt und dieses in die organische Phase aufgenommen. Diese Base wird zweckmässigerweise in stöchiometrischer Menge oder in einem Ueberschuss von bis zu einem 4:1-Molverhältnis bezogen auf die Menge Hydrazinsalz eingesetzt. Anschliessend wird das Benzoesäurefluorid oder -chlorid VI ($R^4$=Fluor bzw. Chlor), gegebenenfalls im verwendeten Lösungsmittel gelöst, zur organischen Phase zugetropft, in der die Reaktion dann

stattfindet. Die freiwerdende Säure wird dabei in die wässrige Phase aufgenommen. Die Isolierung des Hydrazins II erfolgt zweckmässigerweise durch Abtrennen der organischen Phase, gefolgt vom Entfernen, z.B. mittels Destillierens, des organischen Lösungsmittels.

Im allgemeinen liegen die Reaktionstemperaturen zwischen -20°C und dem Siedepunkt des verwendeten Verdünnungsmittels, vorzugsweise zwischen 0°C und 80°C, bzw. bei Verwendung des Zweiphasensystems zwischen 5°C und ca. 30°C. Vorzugsweise wird im letzteren Fall bei Raumtemperatur gearbeitet.

Die als Ausgangsmaterialien in der Verfahrensvariante a) verwendeten Hydrazide der Formel II, in denen X' Schwefel bedeutet, können dadurch hergestellt werden, dass man ein Hydrazid der Formel II, in der X' Sauerstoff bedeutet, mit einem Schwefelungsmittel, wie dem oben erwähnten Lawesson--Reagens in Toluol, behandelt, und zwar zweckmässigerweise unter den im Zusammenhang mit der Verfahrensvariante c) oben erwähnten Reaktionsbedingungen.

Eine weitere Methode zur Herstellung dieser Hydrazide besteht darin, dass man ein Benzhydrazinoylchlorid der allgemeinen Formel

$$R^1-NH-N=C-R^2$$
$$\quad\quad\quad\quad |$$
$$\quad\quad\quad\quad Cl$$

VII

worin $R^1$ und $R^2$ die oben angegebenen Bedeutungen besitzen,

mit Schwefelwasserstoff in Gegenwart einer Base, z.B. Triäthylamin, versetzt. Dieses Verfahren ist u.a. in Can. J. Chem., 52, 879 (1974) näher beschrieben.

Die als Ausgangsmaterialien der Verfahrensvariante b)

verwendeten 2,4-Dihydro-3H-1,2,4-triazol-3-one der Formel III sind zum Teil aus der europäischen Patentpublikation Nr. 208.321 bekannt geworden und können z.B. gemäss den nachfolgenden Reaktionsschemata 1 und 2 hergestellt werden:

Reaktionsschema 1

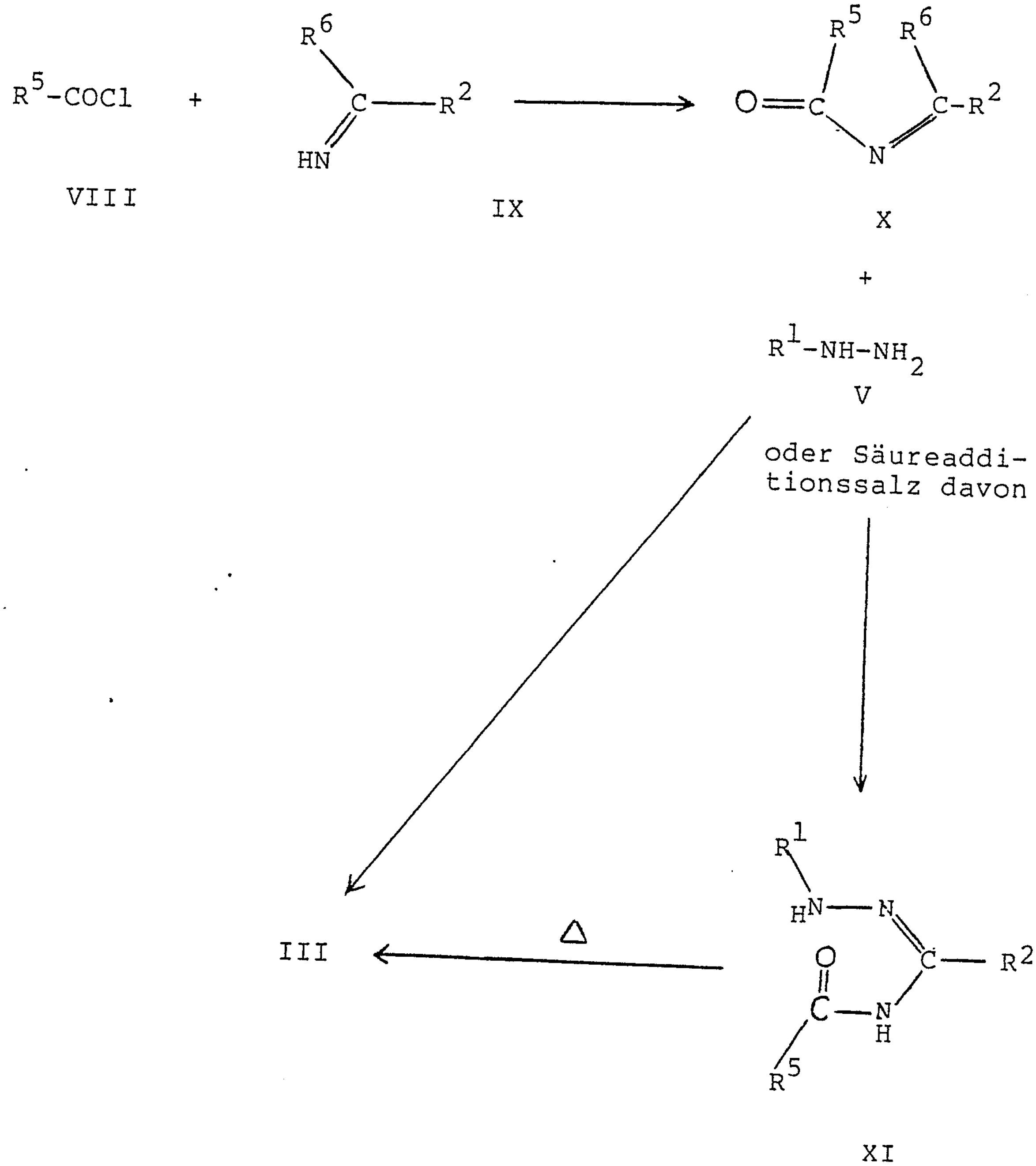


VIII IX X V XI III

Reaktionsschema 2

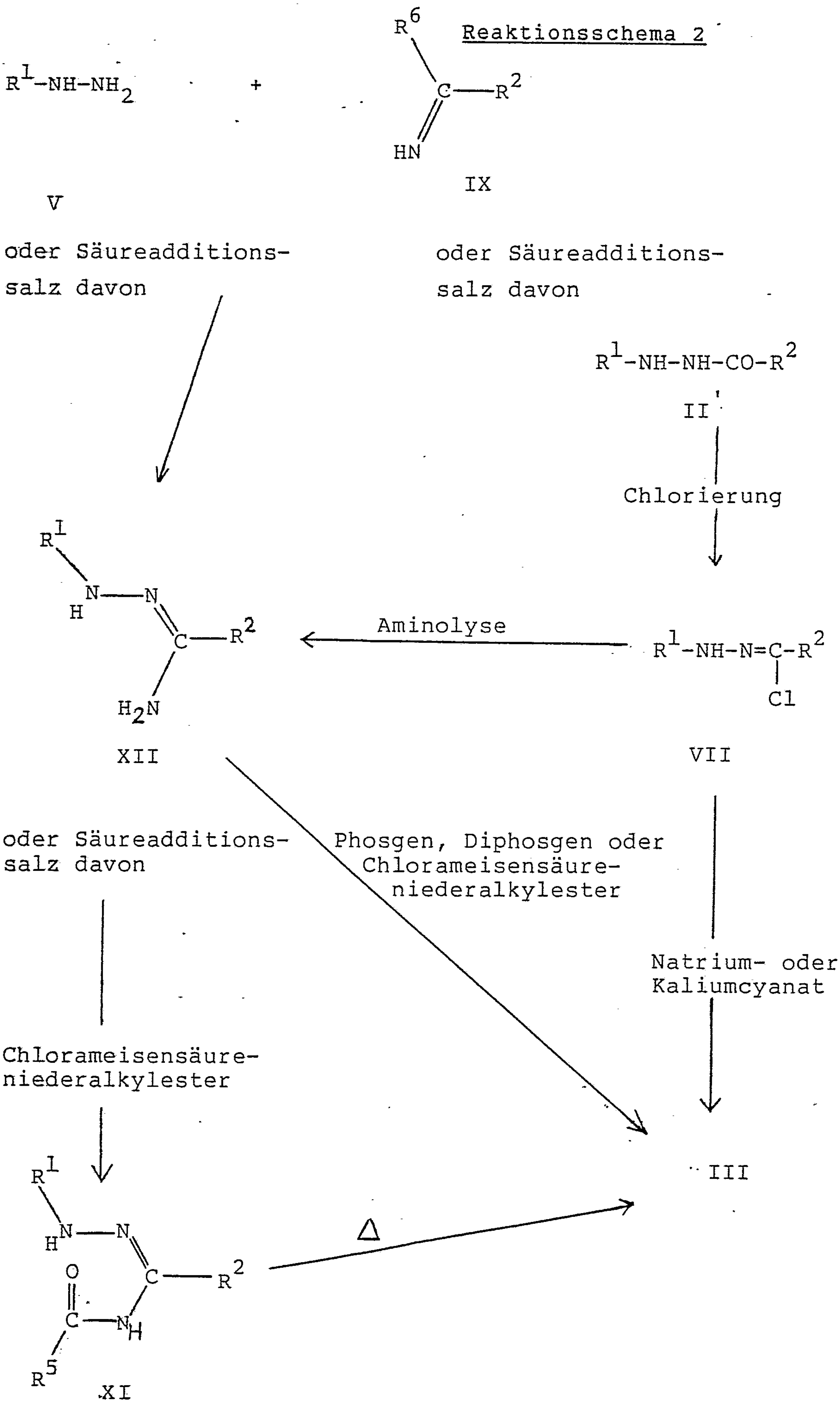

In den Reaktionsschemata besitzen $R^1$ und $R^2$ die oben angegebenen Bedeutungen; $R^5$ und $R^6$ bedeuten nieder Alkoxy, insbesondere $C_{1-3}$-Alkoxy, wie Methoxy oder Aethoxy.

Die erste Methode zur Herstellung der Ausgangsmaterialien III (Reaktionsschema 1) besteht darin, einen Chlorameisensäure-niederalkylester der Formel VIII mit einem Benzimidsäure-alkylester der Formel IX umzusetzen und den so hergestellten N-Alkoxycarbonyl-benzimidsäureester der Formel X mit einem Hydrazin der Formel V oder mit einem Säureadditionssalz davon zur Reaktion zu bringen. Die erste Stufe erfolgt zweckmässigerweise in Gegenwart einer Base, insbesondere einer sterisch gehinderten Base, wie 2,6-Lutidin oder sym-Collidin, sowie in Gegenwart eines inerten Verdünnungsmittels, wie eines Kohlenwasserstoffes, z.B. n-Heptan oder Petroleumbenzin, bei erhöhter Temperatur, z.B. bei der Rückflusstemperatur des Reaktionsgemisches. Dieses Verfahren ist beispielsweise in Synthesis 1983, 483-6 exemplifiziert. Die Umsetzung der Verbindung der Formel X mit dem Hydrazin der Formel V oder mit einem Säureadditionssalz davon, z.B. einem Salz mit einer Mineralsäure, wie Salzsäure oder Schwefelsäure, oder mit einer organischen Säure, wie Oxalsäure, erfolgt zweckmässigerweise in einem inerten Verdünnungsmittel, wie einem chlorierten aliphatischen oder aromatischen Kohlenwasserstoff, z.B. Tetrachlorkohlenstoff, 1,1,2-Trichloräthan oder 1,2-Dichlorbenzol; einem Alkohol, z.B. Aethanol oder Methylcellosolve; einem aliphatischen oder cyclischen Aether, z.B. Diäthylenglykol-diäthyläther, Tetrahydrofuran oder Dioxan; einem aliphatischen Nitril, z.B. Acetonitril; einem aliphatischen oder aromatischen Kohlenwasserstoff, z.B. n-Heptan, Toluol oder o- oder p-Xylol; oder Dimethylformamid. Bei Verwendung eines Mineralsalzes oder eines organischen Salzes des Hydrazins der Formel V wird vorzugsweise auch noch in Gegenwart eines säurebindenden Mittels, z.B. Triäthylamin, 6-Aethyl-2-methylpyridin, 2,6-Lutidin oder Natriumacetat, umgesetzt. Die Reaktionstemperaturen können in einem grossen Bereich

variiert werden, wobei im allgemeinen bei Temperaturen zwischen 20°C und der Rückflusstemperatur des Reaktionsgemisches, vorzugsweise zwischen 80°C und 120°C, gearbeitet wird.

Gemäss Schema 1 kann aber vorerst auch nur eine Hydrazinaddition stattfinden, und zwar unter Bildung einer Verbindung der Formel XI. Die Verbindung kann nach Isolierung durch Erhitzen auf ca. 140-220°C (in der Schmelze) unter Abspaltung des niederen Alkanols $R^5H$ in das entsprechende 2,4- oder 1,2-Dihydro-3H-1,2,4-triazol-3-on der Formel III bzw. III'

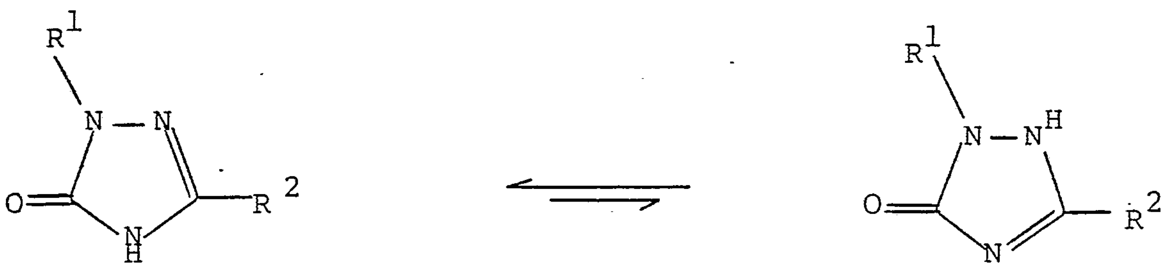

III III'

übergeführt werden. Die Isolierung der Verbindung XI ist jedoch nicht erforderlich: Falls man der N-Alkoxycarbonyl--benzimidsäureester X mit dem Hydrazin V in einem gegebenenfalls chlorierten aromatischen Kohlenwasserstoff, wie Toluol oder 1,2-Dichlorbenzol, bei Temperaturen zwischen 0°C und 80°C umsetzt, und man anschliessend auf ca. 110-160°C erhitzt, bildet sich die Verbindung XI. Zweckmässigerweise wird dabei das gebildete Alkanol $R^5H$ aus dem Reaktionsgemisch azeotrop abdestilliert.

Bei der zweiten Methode zur Herstellung der Ausgangsmaterialien III (Reaktionsschema 2) handelt es sich in der wesentlichen Verfahrensstufe um die Cyclisierung des Amidrazons der Formel XII oder eines Säureadditionssalzes davon, z.B. des Hydrochlorids, mit Phosgen, Diphosgen oder einem Chlorameisensäure-niederalkylester (vorzugsweise einem $C_{1-3}$-Alkylester), und zwar zweckmässigerweise in Gegenwart

eines inerten Verdünnungsmittels und unter Verwendung eines säurebindenden Mittels. Als Verdünnungsmittel eignen sich insbesondere chlorierte aliphatische oder aromatische Kohlenwasserstoffe, z.B. 1,1,2-Trichloräthan und 1,2-Dichlorbenzol; aliphatische oder cyclische Aether, z.B. Diäthylenglykol-diäthyläther, Tetrahydrofuran und Dioxan; aliphatische Nitrile, z.B. Acetonitril; aliphatische oder aromatische Kohlenwasserstoffe, z.B. Toluol und o- und/oder p-Xylol; und Pyridin. Vorzugsweise wird ein tertiäres Amin, wie Triäthylamin oder Pyridin, als säurebindendes Mittel benützt. Die Reaktionstemperaturen können in einem grossen Bereich variiert werden, wobei zweckmässigerweise bei Temperaturen zwischen 0°C und der Rückflusstemperatur des Reaktionsgemisches gearbeitet wird.

Bei dieser Methode findet gelegentlich, insbesondere bei Verwendung eines Chlorameisensäure-niederalkylesters, vorerst nur Carbamoylierung statt, wobei zunächst eine Verbindung der Formel XI, in der $R^5$ nieder Alkoxy bedeutet, gebildet wird. Diese Verbindung kann gegebenenfalls nach Isolierung in das entsprechende 2,4-Dihydro-3H-1,2,4--triazol-3-on der Formel III übergeführt werden, und zwar unter den Reaktionsbedingungen des Schemas 1.

Eine weitere Methode zur Herstellung der Ausgangsmaterialien III besteht darin, ein Benzhydrazinoylchlorid der Formel VII mit Natrium- oder Kaliumcyanat umzusetzen [siehe Reaktionsschema 2 sowie Gazz. Chim. Ital. <u>68</u>, 147 (1968)]. Die Umsetzung erfolgt zweckmässigerweise in einem inerten Verdünnungsmittel, wie Aethanol, Aceton, Acetonitril, Essigsäure, Tetrahydrofuran, Dimethylformamid, Wasser oder einem Gemisch von zwei oder mehreren dieser Verdünnungsmittel, z.B. einem Aethanol/Wasser- oder Aceton/Wasser-Gemisch. In solchen Gemischen ist vorzugsweise ca. 5 bis 25 Volumenprozent Wasser vorhanden. Die Reaktionstemperaturen können in einem grossen Bereich variiert werden, und zwar von ca. -10°C bis zur Rückflusstemperatur des Reaktionsgemisches.

Das Natrium- oder Kaliumcyanat wird dabei in stöchiometrischer Menge oder in einem geringen Ueberschuss, und zwar in bis zu einem 3:1-Molverhältnis bezogen auf die Menge Benzhydrazinoylchlorid VII, eingesetzt.

Die Amidrazone XII selber und deren Säureadditionssalze sind entweder bekannt oder können in an sich bekannter Weise hergestellt werden, beispielsweise dadurch, dass man ein Hydrazin der Formel V oder ein Säureadditionssalz davon, z.B. das Hydrochlorid, mit einem Benzimidsäure-alkylester der Formel IX oder mit einem Säureadditionssalz davon, z.B. dem Hydrochlorid- oder Tetrafluorboratsalz, umsetzt. Die Umsetzung erfolgt gegebenenfalls in einem inerten Verdünnungsmittel, wie einem chlorierten aliphatischen Kohlenwasserstoff, z.B. Methylenchlorid; einem Aromaten, z.B. Toluol; einem aliphatischen oder cyclischen Aether, z.B. Tetrahydrofuran oder Dioxan; einem niederen Alkanol, z.B. Aethanol; oder Pyridin, bei relativ niedrigen Temperaturen, z.B. zwischen 0°C und ca. 60°C. Vorteilhaft werden dabei die Ausgangsmaterialien der Formeln V und IX in stöchiometrischer Menge eingesetzt.

Eine weitere Methode zur Herstellung dieser Amidrazone besteht darin, dass man ein Hydrazid der Formel II' mit Phosphoroxychlorid gegebenenfalls in geringem Ueberschuss, also mit ca. 1,005 bis 4, besonders bevorzugt mit ca. 1,02 bis 1,15 Aequivalenten Phosphoroxychlorid pro Aequivalent Hydrazid, behandelt, und zwar bei Temperaturen zwischen 85°C und 110°C und gegebenenfalls in Gegenwart eines Verdünnungsmittels, wie beispielsweise Toluol, Dioxan oder 1,2-Dichloräthan, besonders bevorzugt bei 100°C in Gegenwart von Toluol, um zu dem entsprechenden Benzhydrazinoylchlorid der Formel VII zu gelangen, und dies dann einer Aminolyse unterwirft. Dazu wird beispielsweise eine Lösung des Benzhydrazinoylchlorids in einem inerten Lösungsmittel, wie einem Aromaten, z.B. Toluol, oder einem aliphatischen oder cyclischen Aether, z.B. Diäthyläther oder Dioxan, vorgelegt

und bei ca. -40°C bis 20°C mit einer Lösung von Ammoniak in Aethanol oder Wasser bzw. mit gasförmigem Ammoniak behandelt. Besonders bevorzugt wird das Reaktionsgemisch der Chlorierung direkt in ein gut gekühltes Gemisch von wässrigem Ammoniak in Dioxan oder Aethanol, oder vom trockenen Ammoniak in Diäthyläther oder Aethanol eingetragen und alsdann das Produkt der Formel XII durch Extraktion mit einer geeigneten Säure, z.B. Salzsäure, als entsprechendes Salz in wässriger Lösung abgetrennt. Durch Zugabe einer Base, z.B. Natronlauge, lässt sich das Amidrazon XII in reinem Zustand isolieren. Bei dieser Prozedur ist es vorteilhaft, die Aminolyse bei tiefen Temperaturen, z.B. zwischen -50°C und 10°C, vorzugsweise zwischen -20°C und 5°C, durchzuführen, insbesondere deshalb, weil sonst das Benzhydrazinoylchlorid VII zur Dimerisierung neigt.

Als Alternative zur unmittelbaren Behandlung des Reaktionsgemisches der Chlorierung mit Ammoniak kann man dieses Reaktionsgemisch in Wasser aufnehmen und unter gutem Rühren das Benzhydrazinoylchlorid VII mit einem geeigneten organischen Lösungsmittel aus der wässrigen Phase extrahieren. Dabei wird überschüssiges Chlorierungsmittel entfernt. Als organische Lösungsmittel zu diesem Zwecke eignen sich aliphatische Aether, z.B. Diäthyläther und tert.Butylmethyläther, chlorierte aliphatische Kohlenwasserstoffe, z.B. Methylenchlorid, und aliphatische oder aromatische Kohlenwasserstoffe, z.B. n-Hexan und Toluol. Vorzugsweise werden jedoch wässrig-organische Gemische, z.B. Toluol/Wasser-Gemische, verwendet. Gegebenenfalls kann vor dem Extrahieren die wässrige Phase durch Zusatz einer Base auf einen pH-Wert im Bereich von 2 bis 7 eingestellt werden. Nach erfolgtem Extrahieren kann das Benzhydrazinoylchlorid aus dessen Lösung im organischen Lösungsmittel isoliert und der anschliessenden Aminolyse zum Amidrazon XII bzw. der direkten Umsetzung mit Natrium- oder Kaliumcyanat zum 2,4-Dihydro-3H-1,2,4-triazol-3-on III unterworfen werden.

Die als Ausgangsmaterialien der Verfahrensvariante c) verwendeten Verbindungen der Formel I' sind als Untergruppe der Verbindungen der Formel I gemäss Verfahrensvariante a) (X= Sauerstoff oder Schwefel) bzw. b) (X= $NR^3$) zugänglich.

Die restlichen Ausgangsmaterialien bzw. Reagenzien, also u.a. die Verbindungen der Formeln V (und deren Säureadditionssalze), VI, VIII und IX, sind entweder bekannt oder können nach an sich bekannten Methoden hergestellt werden. Die Isolierung und die Reinigung der so hergestellten Ausgangsmaterialien können ebenfalls in an sich bekannter Weise erfolgen.

Die erfindungsgemässen Verbindungen, d.h. die Verbindungen der Formel I, sind ganz allgemein als Pestizide von Wert. Sie erweisen sich als besonders wertvoll zur Bekämpfung von Insekten und Milben, insbesondere von

- Homoptera (insbesondere Blattläusen), wie z.B.
  Aphis fabae, Aphis gossypii, Aphis pomi;
  Acyrthosiphon pisum, Acyrthosiphon dirhodum;
  Brevicoryne brassicae;
  Dysaphis devecta, Dysaphis pyri, Dysaphis plantaginea;
  Macrosiphum rosae; Macrosiphum avenae;
  Myzus persicae, Myzus cerasi;
  Phorodon humuli;
  Rhopalosiphum insertum, Rhopalosiphum padi;
  Toxoptera aurantii;
  Nasonovia ribisnigri;
  Hyalopterus pruni;

- Blattflöhen (Psyllina), wie z.B. Psylla piri, Psylla pirisuga, Psylla piricola, Psylla mali; Trioza apicalis;

- Weissen Fliegen, wie z.B. Trialeurodes vaporariorum; Aleurothrixus floccosus; Bemisia tabaci; Aleurodes proletella; Aleurocanthus woglumi; Dialeurodes citri;

- Milben, die bei dem Pflanzenschutz von Bedeutung sind, wie z.B. Tetranychidae (Spinnmilben), insbesondere Tetranychus urticae, Tetranychus cinnabarinus, Tetranychus turkestani, Tetranychus McDanieli, Tetranychus kanzawai; Panonychus ulmi, Panonychus citri; Phyllocoptruta oleivora; Aculus schlechtendali; Phyllocoptes vitis; Aceria essigi, Aceria gracilis; Cecidophyopsis ribis; Eriophyes vitis; Eotetranychus sexmaculatus, Eotetranychus carpini; Hemitarsonemus latus;

- Milben, die in der Veterinärmedizin von Bedeutung sind, wie z.B. Macronyssus bursa, Macronyssus sylviarum, Macronyssus lacoti; Dermanyssus gallinae;

- Zecken, insbesondere der Familien Ixodidae und Argasidae und der Ordnungen Boophilus, Amblyomma, Hyalomma, Rhipicephalus, Ixodes, Argas und Ornithodorus.

Die erfindungsgemässen Verbindungen wirken als Kontakt- und Frassgifte. Zudem werden einige der Verbindungen von verschiedenen Pflanzen aufgenommen, so dass die zu bekämpfenden Schädlinge beim Fressen der Pflanzen vernichtet werden. Diese Verbindungen weisen also systemische Wirkung auf.

Das erfindungsgemässe Schädlingsbekämpfungsmittel ist dadurch gekennzeichnet, dass es eine wirksame Menge mindestens einer Verbindung der allgemeinen Formel I, wie oben definiert, sowie Formulierungshilfsstoffe enthält. Das Mittel enthält zweckmässigerweise zumindest einen der folgenden Formulierungshilfsstoffe:

Feste Trägerstoffe; Lösungs- bzw. Dispersionsmittel; Tenside (Netz- und Emulgiermittel); Dispergatoren (ohne Tensidwirkung); und Stabilisatoren.

Unter Verwendung solcher und anderer Hilfsstoffe können die Verbindungen der Formel I in die üblichen Formulierungen übergeführt werden, wie Lösungen, Suspensionen, Emulsionen, emulgierbare Konzentrate, Pasten, Schäume, Stäube, Pulver und Granulate.

Als feste Trägerstoffe kommen im wesentlichen in Frage: natürliche Mineralstoffe, wie Kaolin, Tonerden, Kieselgur, Talkum, Bentonit, Kreide, Kalkstein, Quarz, Dolomit, Attapulgit, Montmorillonit und Diatomeenerde; synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Stäube, Pulver oder Granulate vorliegen können.

Als Lösungs- bzw. Dispersionsmittel kommen im wesentlichen in Frage: Aromaten, wie Toluol, Xylole, Benzol und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methylisobutylketon und Cyclohexanon; und stark polare Lösungsmittel, wie Dimethylformamid, N-Methylpyrrolidon und Dimethylsulfoxid, wobei solche

Lösungs- bzw. Dispersionsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol--Treibgase, wie Halogenkohlenwasserstoffe, z.B. Dichlordifluormethan. Im Falle der Benutzung von Wasser als Lösungsmittel können z.B. auch organische Lösungsmittel als Hilfslösungsmittel verwendet werden.

Die Tenside (Netz- und Emulgiermittel) können nicht--ionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-Dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze

von Ligninsulfonsäure, Natriumsalze von Maleinsäureanhydrid--Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel z.B. Epichlorhydrin, Phenylglycidäther und Soyaepoxide; Antioxidantien z.B. Gallussäureester und Butylhydroxytoluol; UV-Absorber z.B. substituierte Benzophenone, Diphenylacrylnitrilsäureester und Zimtsäureester; und Deaktivatoren z.B. Salze der Aethylendiaminotetraessigsäure und Polyglykole.

Die erfindungsgemässen Schädlingsbekämpfungsmittel können neben den Wirkstoffen der Formel I auch andere Wirkstoffe enthalten, z.B. anderweitige Schädlingsbekämpfungsmittel, Schädlingslockstoffe, Fungizide, Bakterizide, Herbizide, Pflanzenwachstumsregulatoren und Düngemittel. Solche Kombinationsmittel eignen sich zur Verstärkung der Aktivität bzw. zur Verbreiterung des Wirkungsspektrums. Gegebenenfalls können dadurch auch Unzulänglichkeiten von bisher bekannten zugesetzten Mitteln ausgeglichen werden.

Es wurde gefunden, dass die erfindungsgemässen Verbindungen, insbesondere die besonders bevorzugten Verbindungen, mit Vorteil in Kombination mit anderen Akariziden, vor allem mit zur Bekämpfung von mobilen Stadien von Milben geeigneten, eingesetzt werden. Beispiele derartiger Akarizide sind Amitraz, Avermectin, Benzoximate, Bromopropylate, Chlorobenzilate, Cyhexatin, Dicofol, Fenbutatin oxide, Methidathion, Propargite und Aethyl-5,7-dihydro-6H-dibenz-

[c,e]azepin-6-carboximidat sowie Pyrethroide mit akarizider Wirkung, wie beispielsweise Fluvalinate, Biphenthrin und Cyano-3-phenoxybenzyl-3-(2-chlor-2,3,3-trifluorprop-1-enyl)--2,2-dimethyl-cyclopropancarboxylat. Die Anwendung kann als Gemisch oder getrennt erfolgen. Dabei können die erfindungsgemässen Wirkstoffe den Nachteil der bekannten Akarizide mit Wirkungsschwerpunkt gegen adulte Schädlinge egalisieren, indem die nach Einsatz der bekannten Akarizide überlebenden Eier und Larven, welche sich rasch zu einer neuen schädlichen Population entwickeln können, ebenfalls abgetötet werden.

Die erfindungsgemässen Schädlingsbekämpfungsmittel enthalten im allgemeinen zwischen 0,005 und 95 Gewichtsprozent der erfindungsgemässen Verbindung(en) der Formel I als Wirkstoff(e). Sie können in einer Form vorliegen, die sich für die Lagerung und den Transport eignet. In solchen Formen, z.B. emulgierbaren Konzentraten, ist die Wirkstoffkonzentration normalerweise im höheren Bereich der obigen Konzentrationsreihe. Diese Formen können dann mit gleichen oder verschiedenen Formulierungshilfsstoffen bis zur Wirkstoffkonzentrationen verdünnt werden, die sich für den praktischen Gebrauch eignen, und solche Konzentrationen liegen normalerweise im niedrigeren Bereich der obigen Konzentrationsreihe. Emulgierbare Konzentrate enthalten im allgemeinen 5 bis 95 Gewichtsprozent, vorzugsweise 10 bis 80 Gewichtsprozent, der erfindungsgemässen Verbindung(en). Als Anwendungsformen kommen u.a. gebrauchsfertige Lösungen, Emulsionen, Suspensionen, Schäume, Pulver, Pasten, Stäubemittel und Granulate in Frage. Die Wirkstoffkonzentrationen in solchen anwendungsfertigen Zubereitungen können in grossen Bereichen variiert werden. In Spritzbrühen können z.B. Konzentrationen zwischen 0,005 und 0,5 Gewichtsprozent vorliegen. Im Ultra-Low-Volume-Verfahren können Spritzbrühen formuliert werden, in denen die Wirkstoffkonzentration vorzugsweise von 10 bis 20 Gewichsprozent beträgt, während die im Low-Volume-Verfahren und im High-

-Volume-Verfahren formulierten Spritzbrühen vorzugsweise eine Wirkstoffkonzentration von 0,01 bis 0,5 bzw. 0,005 bis 0,1 Gewichtsprozent aufweisen. Granulate enthalten vorzugsweise von 5 bis 50 Gewichtsprozent der erfindungsgemässen Verbindung(en) als Wirkstoff.

Die erfindungsgemässen Schädlingsbekämpfungsmittel können dadurch hergestellt werden, dass man mindestens eine Verbindung der allgemeinen Formel I mit Formulierungshilfsstoffen vermischt.

Die Herstellung der Mittel kann in bekannter Weise durchgeführt werden, z.B. durch Vermischen des Wirkstoffes mit festen Trägerstoffen, durch Auflösem oder Suspendieren in geeigneten Lösungs- bzw. Dispersionsmitteln, eventuell unter Verwendung von Tensiden als Netz- oder Emulgiermitteln oder von Dispergatoren, durch Verdünnen bereits vorbereiteter emulgierbarer Konzentrate mit Lösungs- bzw. Dispersionsmitteln, usw.

Im Falle von pulverförmigen Mitteln kann der Wirkstoff mit einem festen Trägerstoff vermischt werden, z.B. durch Zusammenmahlen; oder man kann den festen Trägerstoff mit einer Lösung oder Suspension des Wirkstoffes imprägnieren und dann das Lösungs- oder Suspensionsmittel durch Abdunsten, Erhitzen oder durch Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die Verbindungen der Formel I können auch mit einem Tensid und einem festen Trägerstoff zur Bildung eines netzbaren Pulvers vermischt werden, welches in Wasser dispergierbar ist, oder sie können mit einem festen vorgranulierten Trägerstoff zur Bildung eines granulatförmigen Produktes vermischt werden.

Wenn gewünscht, kann die Verbindung der Formel I in einem mit Wasser nicht mischbaren Lösungsmittel, wie beispielsweise einem alicyclischen Keton, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach die Lösung mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Das erfindungsgemässe Verfahren zur Bekämpfung von Schädlingen ist dadurch gekennzeichnet, dass man das zu schützende Gut oder die Schädlinge selbst mit einer wirksamen Menge einer erfindungsgemässen Verbindung bzw. eines erfindungsgemässen Schädlingsbekämpfungsmittels behandelt. Dieses Anwendungsverfahren kann durch Boden- oder Blattapplikation, bzw. durch Applikation auf die zu schützenden Tiere, Vorräte oder Materialien, je nach Art der zu bekämpfenden Schädlinge, durchgeführt werden. Die Bekämpfung wird beispielsweise durch Kontakt oder durch Einnahme mit der Nahrung erzielt.

Die Anwendung kann in konventioneller Weise geschehen, z.B. durch Verspritzen, Versprühen, Vernebeln, Verstäuben, Verstreuen, Eindrillen, Verräuchern, Giessen, Beizen oder Inkrustieren. Pulverförmige Präparate können z.B. als Stäubemittel mit Hilfe der üblichen Verstäubegeräte auf die Schädlinge bzw. auf das zu schützende Gut, z.B. Pflanzen oder Tiere, aufgebracht werden. Wässrige Suspensionen sind z.B. als Spritzmittel anwendbar.

Bei der Anwendung im Pflanzenschutz genügt in der Regel eine Dosierung von ca. 50-500 g Wirkstoff [Verbindungen(en)

der Formel I]/ha, z.B. wie dies bei der Applikation von 2000 l einer Spritzbrühe, die 0,0025-0,025 Gewichtsprozent Wirkstoff enthält, auf 1 ha bepflanzten Erdbodens der Fall ist.

Die nachfolgenden Beispiele dienen zur näheren Erläuterung der Erfindung.

I. Herstellung der Wirkstoffe der Formel I:

Beispiel 1

2,5 g (7,5 mMol) 2-Chlor-6-fluor-N'-(α,α,α-trifluor-o-tolyl)-benzhydrazid werden in 35 ml einer 20%-igen Lösung von Phosgen (67 mMol) in Toluol während 16 Stunden auf Rückflusstemperatur (Innentemperatur 80°C) erhitzt. Ueberschüssiges Phosgen und Toluol werden dann abdestilliert, und der Rückstand wird in 30 ml Methylenchlorid gelöst und unter Zusatz von 6,7 ml (48 mMol) Triäthylamin 30 Minuten auf Rückflusstemperatur erhitzt. Anschliessend wird mit Wasser gewaschen, über wasserfreiem Natriumsulfat getrocknet und unter vermindertem Druck eingedampft. Nach chromatograhischer Reinigung des Rohproduktes mit Toluol/n-Hexan (1:1) als Laufmittel und Umkristallisieren aus Methylenchlorid/n-Hexan erhält man reines 5-(2-Chlor-6--fluorphenyl)-3-(α,α,α-trifluor-o-tolyl) -1,3,4-oxadiazol-2(3H)-on, Smp. 109-109,5°C; IR-Spektrum: 1792 $cm^{-1}$; Massenspektrum: 358(14), 314(23), 159(100).

Beispiel 2

39 g (0,13 Mol) 2-Chlor-6-fluor-N'-(o-chlorphenyl)--benzhydrazid werden mit einer Lösung von 40 g (0,40 Mol) Phosgen in 275 ml Toluol versetzt, und das Gemisch wird 2,5 Stunden auf Rückflusstemperatur (Innentemperatur 59°C) erhitzt. Man gibt dann weitere 13 g (0,13 Mol) Phosgen in 70 ml Toluol zu und erhitzt das Reaktionsgemisch noch

2,5 Stunden auf Rückflusstemperatur. Das überschüssige Phosgen und Toluol werden abdestilliert, und man nimmt den Rückstand in 500 ml Diäthyläther auf, wäscht die Lösung mit halbgesättigter Natriumchloridlösung, trocknet sie über wasserfreiem Magnesiumsulfat und dampft sie unter vermindertem Druck ein. Nach Umkristallisieren des Rohproduktes aus einem Gemisch von Diäthyläther und n-Hexan erhält man reines 5-(2-Chlor-6-fluorphenyl)-3-(2-chlorphenyl)-1,3,4-oxadiazol-2(3H)-on, Smp. 92-95°C; IR-Spektrum: 1790 $cm^{-1}$; Massenspektrum: 324(12), 280(10), 125(100).

## Beispiel 3

Zu einem Gemisch von 5,34 g (16 mMol) 2,6-Difluor-N'-(o-bromphenyl)-benzhydrazid und 5,50 ml (40 mMol) Triäthylamin in 35 ml Dioxan werden bei 10-20°C 2,3 ml (19 mMol) Chlorameisensäure-trichlormethylester zugetropft. Man erhitzt das Reaktionsgemisch noch 3 Stunden bei Rückflusstemperatur und destilliert anschliessend das Lösungsmittel und überschüssiges Reagens unter vermindertem Druck ab. Der Rückstand wird in Diäthyläther aufgenommen und die Lösung je einmal mit Wasser und Natriumhydrogencarbonatlösung gewaschen, über wasserfreiem Natriumsulfat getrocknet und unter vermindertem Druck eingedampft. Nach Umkristallisieren des Rückstandes aus Aceton/n-Hexan erhält man reines 3-(2-Bromphenyl)-5-(2,6-difluorphenyl)-1,3,4-oxadiazol-2(3H)-on, Smp. 111-114°C; IR-Spektrum: 1790 $cm^{-1}$; Massenspektrum: 354/352(15), 310/308(7), 171/169(41), 90(100).

## Beispiel 4

Zu einem Gemisch von 4,28 g (14 mMol) 2-Chlor-N'-(α,α,α-trifluor-o-tolyl)-benzhydrazid und 2,7 ml (19 mMol) Triäthylamin in 30 ml Toluol werden unter Kühlung 1,8 ml (19 mMol) Chlorameisensäure-äthylester zugetropft. Man erhitzt das Reaktionsgemisch noch 4 Stunden bei Rückflusstemperatur und schüttelt es dann mit Diäthyläther gegen

Wasser und Natriumhydrogencarbonatlösung aus. Die organische Phase wird über wasserfreiem Magnesiumsulfat getrocknet und unter vermindertem Druck eingedampft. Dann wird das zurückbleibende Oel unter Verwendung von Aethylacetat/n-Hexan (1:4) als Laufmittel chromatographisch gereinigt. Auf diese Weise erhält man den 3-(2-Chlorbenzoyl)-2-(α,α,α-trifluor-o-tolyl)-carbazinsäure-äthylester als fast farbloses Oel, IR-Spektrum ($CHCl_3$): 3405, 1735, 1710 $cm^{-1}$; $^1H$-NMR ($CDCl_3$): 4,28 (q,$OC\underline{H}_2CH_3$).

Das obige Produkt wird unter Zusatz von 0,14 g (1,1 mMol) 4-Dimethylaminopyridin ca. 16 Stunden mittels eines Oelbads bei 180°C erhitzt. Nach Kühlen wird das Reaktionsgemisch in Diäthyläther aufgenommen und die Lösung je einmal mit verdünnter wässriger Salzsäurelösung und wässriger Natriumhydrogencarbonatlösung gewaschen, über wasserfreiem Magnesiumsulfat getrocknet und unter vermindertem Druck eingeengt. Man fällt das Produkt durch Zugabe von n-Hexan aus und erhält auf diese Weise das 5-(2-Chlorphenyl)-3-(α,α,α-trifluor-o-tolyl)-1,3,4-oxadiazol-2(3H)-on, Smp. 100-102°C; IR-Spektrum: 1792 $cm^{-1}$; Massenspektrum: 340(39), 296(27), 159(100).

## Beispiele 5-25

Analog dem in Beispiel 1, 2, 3 oder 4 beschriebenen Verfahren werden die entsprechenden Benzhydrazide der Formel II mit Phosgen, Chlorameisensäure-trichlormethylester oder Chlorameisensäure-äthylester umgesetzt, um die in der nachstehenden Tabelle 1 aufgeführten Verbindungen der Formel I, in der Y Sauerstoff bedeutet, herzustellen.

Tabelle 1

| Beispiel | $R^1$ | $R^2$ | X | Physikalische Daten |
|---|---|---|---|---|
| 5 | 2-Chlorphenyl | 2-Chlorphenyl | O | Smp. 113-114°C; IR-Spektrum: 1788 $cm^{-1}$; Massenspektrum: 306(20), 262(13), 125(100). |
| 6 | 2-Methoxyphenyl | 2-Chlor-6-fluorphenyl | O | Smp. 109-110°C; IR-Spektrum: 1788 $cm^{-1}$; Massenspektrum: 320(17), 276(4), 120(100). |
| 7 | α,α,α-Trifluor-m-tolyl | 2,6-Difluorphenyl | O | Smp. 84,5-85,5°C; IR-Spektrum: 1784 $cm^{-1}$; Massenspektrum: 342(41), 298(23), 159(100). |
| 8 | α,α,α-Trifluor-m-tolyl | 2,6-Dimethoxyphenyl | O | Smp. 133-134°C; IR-Spektrum:. 1776 $cm^{-1}$; Massenspektrum: 366(86), 322(69), 163(100), 159(47). |
| 9 | 2-Chlorphenyl | α,α,α-Trifluor-o-tolyl | O | Smp. 98-100°C; IR-Spektrum: 1794 $cm^{-1}$; Massenspektrum: 340(15), 296(11), 125(100). |
| 10 | α,α,α-Trifluor-o-tolyl | 2,6-Difluorphenyl | O | Smp. 115-117°C; IR-Spektrum: 1792 $cm^{-1}$; Massenspektrum: 342(44), 298(31), 159(100). |
| 11 | 2-Chlorphenyl | " | O | Smp. 113-115°C; IR-Spektrum: 1792 $cm^{-1}$; Massenspektrum: 308(24), 264(13), 125(100). |
| 12 | " | 2,6-Dichlorphenyl | O | Smp. 94-95°C; IR-Spektrum: 1790 $cm^{-1}$; Massenspektrum: 340(9), 296(5), 125(100). |
| 13 | " | 2-Chlor-4-fluorphenyl | O | Smp. 126-128°C; IR-Spektrum: 1790 $cm^{-1}$; Massenspektrum: 324(14), 280(9), 125(100). |
| 14 | 3-Chlor-o-tolyl | 2-Chlor-6-fluorphenyl | O | Smp. 164-165,5°C; IR-Spektrum: 1788 $cm^{-1}$; Massenspektrum: 338(34), 294(12), 139(100). |

Tabelle 1 (Fortsetzung)

| Beispiel | $R^1$ | $R^2$ | X | Physikalische Daten |
|---|---|---|---|---|
| 15 | 2-Chlorphenyl | 2-Chlor-6-fluor-phenyl | S | Smp. 128-130°C; IR-Spektrum: 1692, 1668 $cm^{-1}$; Massenspektrum: 340(12), 280(23), 125(100). |
| 16 | 3-Chlor-5-tri-fluormethyl-2-pyridyl | 2,6-Difluorphenyl | O | Smp. 106-108°C. |
| 17 | " | 2-Chlorphenyl | O | Smp. 72-75°C. |
| 18 | α,α,α-Tri-fluor-o-tolyl | 2,6-Difluorphenyl | S | Smp. 117-119°C; IR-Spektrum: 1700, 1676 $cm^{-1}$; Massenspektrum: 358(40), 298(59), 159(100). |
| 19 | 4-Chlor-2-tri-fluormethyl-phenyl | " | O | Smp. 111-113°C; IR-Spektrum: 1792 $cm^{-1}$; Massenspektrum: 376(18), 332(15), 193(100). |
| 20 | 4-Trifluormethoxy-phenyl | " | O | Smp. 76-78°C; IR-Spektrum: 1782 $cm^{-1}$; Massenspektrum: 358(25), 314(6), 175(100). |
| 21 | α,α,α-Tri-fluor-o-tolyl | 2-Chlor-4-fluorphenyl | O | Smp. 107-109°C; IR-Spektrum: 1790 $cm^{-1}$; Massenspektrum: 358(35), 314(19), 159(100). |
| 22 | 2-Chlor-4-tri-fluormethyl-phenyl | 2,6-Difluor-phenyl | O | Smp. 118-120°C; IR-Spektrum: 1790 $cm^{-1}$; Massenspektrum: 376(20), 332(23), 193(100). |
| 23 | 2-Methylthio-5-trifluormethyl-phenyl | " | O | Smp. 127-129°C; IR-Spektrum: 1790-$cm^{-1}$; Massenspektrum: 388(40), 344(3), 204(100). |
| 24 | 2,3-Dimethylphenyl | " | O | Smp. 138-140°C; IR-Spektrum: 1788 $cm^{-1}$; Massenspektrum: 302(49), 258(5), 118(100). |
| 25 | 2-Fluorphenyl | 2-Chlor-6-fluorphenyl | O | Smp. 111-112°C; IR-Spektrum: 1788 $cm^{-1}$; Massenspektrum: 308(15), 264(8), 109(100). |

## Beispiel 26

4,79 g (15 mMol) 2,6-Difluor-N'-(α,α,α-trifluor--o-tolyl)-benzhydrazid in 30 ml Toluol werden mit 1,98 g (17 mMol) Thiophosgen versetzt, und das Reaktionsgemisch wird 5 Stunden bei Rückflusstemperatur erhitzt. Alsdann werden bei 45°C unter Kühlen 4,8 ml (35 mMol) Triäthylamin zugetropft, und nach Abkühlen wird ca. 1 Stunde bei Raumtemperatur nachgerührt. Anschliessend wird das Gemisch in 100 ml tert.Butylmethyläther aufgenommen und die Lösung je einmal mit verdünnter, eiskalter Natronlauge und gesättigter Natriumchloridlösung gewaschen, über wasserfreiem Magnesiumsulfat getrocknet und unter vermindertem Druck eingedampft. Man reinigt den Rückstand chromatographisch mit Aceton/n-Hexan (1:4) als Laufmittel und kristallisiert aus Diisopropyläther/n-Hexan. Auf diese Weise erhält man das 5-(2,6--Difluorphenyl)-3-(α,α,α-trifluor-o-tolyl)-1,3,4--oxadiazol-2(3H)-thion als weisses Kristallisat, Smp. 128-130°C; Massenspektrum: 358(58), 298(15), 289(17), 159(100).

## Beispiel 27

Ein Gemisch von 3,75 g (11,0 mMol) 5-(2,6-Difluorphenyl)-2-(α,α,α-trifluor-o-tolyl) -2,4-dihydro-3H--1,2,4-triazol-3-on, 1,05 g (11,0 mMol) Dimethylsulfat und 3,04 g (22,0 mMol) Kaliumcarbonat in Aceton wird während 16 Stunden bei Rückflusstemperatur gerührt. Man filtriert dann die unlöslichen Anteile ab und unterwirft den eingedampften Rückstand einer Chromatographie an Kieselgel unter Verwendung von Aethylacetat/n-Hexan (1:4) als Laufmittel. Als erste Fraktion isoliert man die apolare Nebenkomponente 3-(2,6-Difluorphenyl)-5-methoxy-1-(α,α,α-trifluor -o-tolyl)-1H-1,2,4-triazol, Smp. 118-119°C; Massenspektrum: 355(75), 298(39), 159(100); $^{1}H$-NMR ($CDCl_3$): 4,13 (s,$OC\underline{H}_3$). Nach weiterem Eluieren mit Aethylacetat/n-Hexan (2:3) und anschliessendem Umkristallisieren aus Diäthyl-

äther/n-Hexan erhält man reines 5-(2,6-Difluorphenyl)-4--methyl-2-(α,α,α-trifluor-o-tolyl)-2,4-dihydro-3H--1,2,4-triazol-3-on, Smp. 98-99°C, IR-Spektrum: 1708 $cm^{-1}$; Massenspektrum: 355(75), 298(32), 159(100); $^1H$-NMR ($CDCl_3$): 3,30 (s,N-C$\underline{H}_3$).

Beispiele 28-31

Analog dem in Beispiel 27 beschriebenen Verfahren werden die entsprechenden 2,4-Dihydro-3H-1,2,4-triazol-3--one der Formel III alkyliert, um die in der nachstehenden Tabelle 2 aufgeführten Verbindungen der Formel I, in der X $NR^3$ und Y Sauerstoff bedeuten, herzustellen. Auch die entsprechenden O-alkylierten Nebenprodukte "(O-$R^3$)" sind angegeben.

Tabelle 2

| Beispiel | $R^1$ | $R^2$ | $R^3$ | Physikalische Daten |
|---|---|---|---|---|
| 28 | 2-Chlorphenyl | 2-Chlorphenyl | Methyl ($N-R^3$) | Smp. 143-145°C; IR-Spektrum: 1712 $cm^{-1}$; $^1H$-NMR($CDCl_3$): 3,30 (s,N-C$\underline{H}_3$). |
| 29 | " | " | 2-Propinyl ($N-R^3$) | Smp. 82-84°C; IR-Spektrum: 3305, 1718 $cm^{-1}$; $^1H$-NMR($CDCl_3$): 2,21 (t,J=2,5Hz,C$\underline{H}$), 4,48 (d,J=2,5Hz, N-C$\underline{H}_2$). |
| 29a | " | " | 2-Propinyl ($O-R^3$) | Smp. 92-94°C; $^1H$-NMR($CDCl_3$): 2,58 (t,J=2,5Hz,C$\underline{H}$), 5,16 (d,J=2,5Hz, OC$\underline{H}_2$). |
| 30 | " | 2-Chlor-6-fluorphenyl | Methyl ($N-R^3$) | Smp. 132-134°C; IR-Spektrum: 1710 $cm^{-1}$; $^1H$-NMR($CDCl_3$): 3,24 (s,N-C$\underline{H}_3$); Massenspektrum: 337(35), 302(91), 125(100). |
| 30a | " | " | Methyl ($O-R^3$) | Smp. 111-112°C; Massenspektrum: 337(45), 302(11), 280(11), 125(100). |
| 31 | α,α,α-Trifluor-o-tolyl | 2,6-Difluor-phenyl | 2-Propinyl ($N-R^3$) | Smp. 145°C; $^1H$-NMR($CDCl_3$): 2,17 (t,J=2,5 Hz, C$\underline{H}$), 4,53 (d,J=2,5 Hz, OC$\underline{H}_2$). |

Beispiel 32

Ein Gemisch von 3,25 g (10 mMol) 5-(2-Chlor-6-fluorphenyl)-3-(2-chlorphenyl) -1,3,4-oxadiazol-2(3H)-on (siehe Beispiel 2) und 2,21 g (5,5 mMol) 2,4-Bis-(4-methoxyphenyl)--1,2-dithioxo -1,3,2,4-dithiaphosphetan (Lawesson-Reagens) in 50 ml Toluol wird während 48 Stunden auf Rückflusstemperatur erhitzt. Das ausgefallene Produkt wird abfiltriert und an Kieselgel mit Aethylacetat/n-Hexan (1:4) als Laufmittel chromatographisch gereinigt. Nach anschliessendem Kristallisieren aus Aceton/n-Hexan erhält man reines 5-(2-Chlor-6--fluorphenyl)-3-(2-chlorphenyl) -1,3,4-oxadiazol-2(3H)--thion, Smp. 112-114°C; Massenspektrum: 340(5), 305(70), 150(100), 125(49).

Beispiel 33

Analog dem in Beispiel 32 beschriebenen Verfahren wird durch Behandlung von 5-(2-Chlor-6-fluorphenyl)-3-(2-chlorphenyl)-1,3,4-thiadiazol-2(3H)-on (siehe Beispiel 15) mit Lawesson-Reagens das 5-(2-Chlor-6-fluorphenyl)-3-(2-chlorphenyl)-1,3,4-thiadiazol-2(3H)-thion hergestellt, Smp. 126-128°C; Massenspektrum: 356(2), 321(100), 280(8), 166(59), 125(69).

II. Herstellung der Ausgangsmaterialien der Formel II:

Beispiel 34

Das als Ausgangsmaterial im Beispiel 2 benötigte 2-Chlor-6-fluor-N'-(o-chlorphenyl)-benzhydrazid kann wie folgt hergestellt werden:

In eine auf 0°C gekühlte Lösung von 52,8 g (0,29 Mol) 2-Chlorphenylhydrazin-hydrochlorid in 290 ml Pyridin werden tropfenweise 56,9 g (0,29 Mol) 2-Chlor-6-fluorbenzoylchlorid so eingetragen, dass die Temperatur der Lösung 5°C nicht

übersteigt. Man rührt die Lösung 16 Stunden bei Raumtemperatur und dampft anschliessend das Lösungsmittel azeotrop mit Toluol unter vermindertem Druck ab. Die resultierende rotbraune Suspension wird in 350 ml Diäthyläther aufgenommen und die Lösung der Reihe nach zweimal mit verdünnter Salzsäure und je einmal mit 5%iger Natriumhydrogencarbonatlösung und mit gesättigter Natriumchloridlösung gewaschen. Die wässrigen Phasen werden mit 350 ml Diäthyläther nachextrahiert und die vereinigten organischen Phasen über wasserfreiem Magnesiumsulfat getrocknet und dann eingedampft. Nach Umkristallisieren des Rückstandes aus Aceton/n-Hexan erhält man das 2-Chlor-6-fluor-N'-(o-chlorphenyl)-benzhydrazid, Smp. 156-158°C; IR-Spektrum: 1692 $cm^{-1}$.

## Beispiel 35

Das als Ausgangsmaterial in den Beispielen 10 und 26 benötigte 2,6-Difluor-N'-(α,α,α-trifluor-o-tolyl)-benzhydrazid kann wie folgt hergestellt werden:

59,8 g (0,28 Mol) o-Trifluormethyl-phenylhydrazin-hydrochlorid werden in einem Zweiphasensystem von 280 ml Essigsäure-äthylester und 85 ml Wasser vorgelegt, und das Ganze wird unter gutem Rühren bei 10-20°C portionenweise mit 84,9 g (0,62 Mol) Kaliumcarbonat behandelt. Nachdem sich eine klare Lösung gebildet hat (15 Minuten), werden in den nächsten 30 Minuten 49,4 g (0,28 Mol) 2,6-Difluorbenzoylchlorid so zugetropft, dass die Temperatur höchstens bei 25°C gehalten wird. Nach weiteren 30 Minuten wird mit 300 ml Wasser verdünnt und die organische Phase abgetrennt, einmal mit gesättigter Natriumchloridlösung gewaschen, über wasserfreiem Natriumsulfat getrocknet und zur Trockene eingedampft. Der Rückstand wird in 30 ml heissem Aceton gelöst und durch Zugabe von 500 ml n-Hexan das 2,6-Difluor-N'-(α,α,α-trifluor-o-tolyl)-benzhydrazid ausgefällt. Smp. 123-125°C; IR-Spektrum: 3405, 1694 $cm^{-1}$.

## Beispiel 36

Das 2,6-Difluor-N'-(α,α,α-trifluor-o-tolyl)-benzhydrazid kann auch wie folgt hergestellt werden:

Zu einer Suspension von 10,4 g (49 mMol) α,α,α-Trifluor-o-tolylhydrazin-hydrochlorid in 50 ml Essigsäure-äthylester gibt man unter Rühren bei 10°C 13,6 g (98 mMol) Kaliumcarbonat in 70 ml Wasser zu. Anschliessend tropft man zur klaren Lösung unter Kühlen 7,8 g (49 mMol) 2,6-Difluorbenzoylfluorid so zu, dass die Innentemperatur bei 3°C gehalten wird. Man rührt 60 Minuten bei 5°C, danach 4 Stunden bei 25°C, und trennt dann die organische Phase ab. Die wässrige Phase wird mit 50 ml Essigsäure-äthylester nachextrahiert und anschliessend zur Fällung des Calciumfluorids mit 1,85 g (25 mMol) Calciumhydroxid versetzt. Die vereinigten organischen Phasen werden je einmal mit verdünnter Salzsäure und Natriumhydrogencarbonatlösung gewaschen, über wasserfreiem Magnesiumsulfat getrocknet und unter vermindertem Druck eingedampft. Nach Kristallisieren aus Aceton und n-Hexan erhält man als weisses Kristallisat das 2,6-Difluor-N'-(α,α,α-trifluor-o-tolyl)-benzhydrazid, Smp. 129-130°C; Massenspektrum m/e: $M^+$ 316(26), 141(100).

## Beispiele 37-57

Analog dem in Beispiel 34 oder 35 beschriebenen Verfahren wird das entsprechende Hydrazin der Formel V in Form des Hydrochlorides mit dem entsprechenden substituierten Benzoesäurechlorid- bzw. fluorid der Formel VI umgesetzt, um die in der nachstehenden Tabelle 3 aufgeführten Hydrazide der Formel II, in der X' Sauerstoff bedeutet, herzustellen. Auch die diesbezüglichen Endprodukte der Formel I werden durch ihre Beispielnummern identifiziert.

Tabelle 3

| Beispiel | Beispiel-Nr. für das Endprodukt der Formel I | $R^1$ | $R^2$ | Physikalische Daten |
|---|---|---|---|---|
| 37 | 1 | α,α,α-Trifluor-o-tolyl | 2-Chlor-6-fluorphenyl | Smp. 148-149°C |
| 38 | 3 | 2-Bromphenyl | 2,6-Difluorphenyl | Smp. 178-180°C |
| 39 | 4 | α,α,α-Trifluor-o-tolyl | 2-Chlorphenyl | Smp. 119-121°C |
| 40 | 5 | 2-Chlorphenyl | 2-Chlorphenyl | Smp. 131-132°C |
| 41 | 6 | 2-Methoxyphenyl | 2-Chlor-6-fluorphenyl | Smp. 138-139°C |
| 42 | 7 | α,α,α-Trifluor-m-tolyl | 2,6-Difluorphenyl | Smp. 150-151°C |
| 43 | 8 | " | 2,6-Dimethoxyphenyl | Smp. 155-156°C |
| 44 | 9 | 2-Chlorphenyl | α,α,α-Trifluor-o-tolyl | Smp. 174-175°C |
| 45 | 11 | " | 2,6-Difluorphenyl | Smp. 177-178°C |
| 46 | 12 | " | 2,6-Dichlorphenyl | Feststoff (nicht gereinigt) |
| 47 | 13 | " | 2-Chlor-4-fluorphenyl | Smp. 122-125°C |

Tabelle 3 (Fortsetzung)

| Beispiel | Beispiel-Nr. für das Endprodukt der Formel I | $R^1$ | $R^2$ | Physikalische Daten |
|---|---|---|---|---|
| 48 | 14 | 3-Chlor-o-tolyl | 2-Chlor-6-fluorphenyl | Smp. 155-157°C |
| 49 | 16 | 3-Chlor-5-trifluor-methyl-2-pyridyl | 2,6-Difluorphenyl | Feststoff (nicht gereinigt) |
| 50 | 17 | " | 2-Chlorphenyl | Feststoff (nicht gereinigt) |
| 51 | 19 | 4-Chlor-2-trifluor-methyl-phenyl | 2,6-Difluorphenyl | Smp. 136-138°C |
| 52 | 20 | 4-Trifluormethoxy-phenyl | " | Smp. 159-161°C |
| 53 | 21 | α,α,α-Tri-fluor-o-tolyl | 2-Chlor-4-fluor-phenyl | Smp. 114-116°C |
| 54 | 22 | 2-Chlor-4-tri-fluormethyl-phenyl | 2,6-Difluorphenyl | Smp. 196-197°C |
| 55 | 23 | 2-Methylthio-5-trifluormethyl-phenyl | " | Smp. 148-150°C |
| 56 | 24 | 2,3-Dimethylphenyl | " | Smp. 154-155°C |
| 57 | 25 | 2-Fluorphenyl | 2-Chlor-6-fluorphenyl | Smp. 116-118°C |

Beispiel 58

Das als Ausgangsmaterial im Beispiel 15 benötigte 2-Chlor-6-fluor-N'-(o-chlorphenyl)-thiobenzhydrazid kann wie folgt hergestellt werden:

9,0 g (30 mMol) 2-Chlor-6-fluor-N'-(o-chlorphenyl)-benzhydrazid (siehe Beispiel 34) werden zusammen mit 6,4 g (15,8 mMol) Lawesson-Reagens während 24 Stunden in 150 ml Toluol auf Rückflusstemperatur erhitzt. Das Reaktionsgemisch wird dann unter Verwendung von Aethylacetat/n-Hexan (1:4) als Laufmittel einer Säulenchromatographie an Kieselgel unterworfen. Die erste Fraktion ergibt eine Kristallmasse, deren IR-Spektrum kein Carbonylsignal aufweist. Man unterwirft diese einer zweiten Chromatographie mit Methylenchlorid/n-Hexan (13:7) und kristallisiert die eingedampfte Fraktion aus Aceton/n-Hexan um. Dabei erhält man als gelbliches Kristallisat reines 2-Chlor-6-fluor-N'-(o-chlorphenyl)-thiobenzhydrazid, Smp. 131-133°C; Mikroanalyse:

| | C % | H % | Cl % | F % | N% | S % |
|---|---|---|---|---|---|---|
| berechnet: | 49,54 | 2,88 | 22,50 | 6,03 | 8,89 | 10,17 |
| gefunden: | 49,51 | 2,93 | 22,58 | 5,64 | 8,86 | 10,37 |

Beispiel 59

Analog dem in Beispiel 58 beschriebenen Verfahren wird durch Behandlung von 2,6-Difluor-N'-(α,α,α-trifluor-o-tolyl)-benzhydrazid (siehe Beispiele 35 und 36) mit Lawesson-Reagens das als Ausgangsmaterial im Beispiel 18 benötigte 2,6-Difluor-N'-(α,α,α-trifluor-o-tolyl)-thiobenzhydrazid hergestellt.

III. Herstellung der Ausgangsmaterialien der Formel III:

Beispiel 60

Das als Ausgangsmaterial in den Beispielen 27 und 31

benötigte 5-(2,6-Difluorphenyl)-2-(α,α,α-trifluor-o--tolyl) -2,4-dihydro-3H-1,2,4-triazol-3-on kann wie folgt hergestellt werden:

Ein Gemisch von 59,7 g (0,19 Mol) 2,6-Difluor-N'--(α,α,α-trifluor-o-tolyl)-benzhydrazid (siehe Beispiele 35 und 36) und 31,8 g (0,21 Mol) Phosphoroxychlorid wird eine Stunde auf Rückflusstemperatur erhitzt. Man nimmt anschliessend das Reaktionsgemisch in 50 ml Dioxan auf und tropft die Lösung, die das in der ersten Reaktionsstufe erzeugte und nicht isolierte 2,6-Difluor-N'--(α,α,α-trifluor-o-tolyl) -benzhydrazinoylchlorid enthält, in ein gut gerührtes Gemisch von 340 ml 25%iger Ammoniak-Lösung und 340 ml Dioxan bei -4°C bis -6°C ein. Dann wird bis zur Phasentrennung mit Diäthyläther verdünnt und die Aetherphase dreimal mit 2N Salzsäure extrahiert. Die saure wässrige Phase wird mit 30%iger Natronlauge alkalisch (pH grösser als 9) gestellt und danach mit Diäthyläther ausgeschüttelt. Nach Trocknen der Aetherphase über wasserfreiem Magnesiumsulfat und Zusatz von n-Hexan erhält man als gelblichen, kristallinen Niederschlag das 2,6-Difluor-N'--(α,α,α-trifluor -o-toluidino)-benzamidrazon, Smp. 108,5-109,5°C.

10 g (32 mMol) des obigen Zwischenproduktes sowie 4,8 ml (35 mMol) Triäthylamin in 60 ml Toluol werden bei 0°C unter Rühren mit einer 1:1-Lösung von 6,0 ml (63 mMol) Chlorameisensäure-äthylester und Toluol versetzt. Man rührt vorerst eine Stunde bei Raumtemperatur und erhitzt dann während ca. 16 Stunden auf Rückflusstemperatur. Das erkaltete Reaktionsgemisch wird abfiltriert und mit Diäthyläther gewaschen. Zur Entfernung von Triäthylammonium-Hydrochlorid wäscht man das Gemisch gut mit Wasser und trocknet den unlöslichen Rückstand bei 80°C/100 mmHg. Auf diese Weise erhält man das 5-(2,6-Difluorphenyl)-2-(α,α,α-trifluor-o-tolyl) -2,4-dihydro-3H-1,2,4-triazol-3-on, Smp. 259-260°C.

## Beispiel 61

Das 5-(2,6-Difluorphenyl)-2-(α,α,α-trifluor-o-tolyl)-2,4-dihydro-3H-1,2,4-triazol-3-on kann auch wie folgt hergestellt werden:

15,8 g (50 mMol) 2,6-Difluor-N'-(α,α,α-trifluor-o-tolyl)-benzhydrazid (siehe Beispiele 35 und 36) werden in 50 ml Toluol suspendiert, und die Suspension wird zusammen mit 8,0 g (52 mMol) Phosphoroxychlorid 5 Stunden bei 100°C erhitzt. Danach verdünnt man das Gemisch mit 150 ml Toluol und versetzt es unter gutem Rühren bei 5°C mit 100 ml Wasser. Die organische Phase wird abgetrennt, mit halbgesättigter Natriumchloridlösung gewaschen, über wasserfreiem Magnesiumsulfat getrocknet und unter vermindertem Druck eingedampft. Man erhält als Oel 15,9 g 2,6-Difluor-N'-(α,α,α-trifluor-o-tolyl)-benzhydrazinoylchlorid, Massenspektrum: 334(56), 298(33), 159(100).

15 g (45 mMol) des obigen Zwischenproduktes werden in 90 ml Aceton gelöst und mit einer Lösung von 5,9 g (90 mMol) Natriumcyanat in 10 ml Wasser versetzt. Man erhitzt das Reaktionsgemisch 20 Minuten bei Rückflusstemperatur, verdünnt es dann mit 270 ml Wasser und lässt es unter Rühren abkühlen. Die entstandenen Kristalle werden abfiltriert, mit Diäthyläther gewaschen und bei 60°C unter vermindertem Druck getrocknet. Man erhält als weisses Kristallisat reines 5-(2,6-Difluorphenyl)-2-(α,α,α-trifluor-o-tolyl)-2,4-dihydro-3H-1,2,4-triazol-3-on, Smp. 273-274°C.

## Beispiel 62 und 63

Analog dem Verfahren der Beispiele 60 und 61 wird das entsprechende Benzhydrazid der Formel II' mit Phosphoroxychlorid behandelt und das resultierende Benzhydrazinoylchlorid der Formel VII entweder einer Aminolyse mit Ammoniak unterworfen und das Produkt der Aminolyse, also das ent-

sprechende Amidrazon der Formel XII oder ein Säureadditionssalz davon mit Chlorameisensäure-äthylester behandelt, oder mit einem Alkalicyanat umgesetzt, um die in der nachstehenden Tabelle 4 aufgeführten Ausgangsmaterialien der Formel III herzustellen. Auch die diesbezüglichen Endprodukte der Formel I werden durch ihre Beispielnummern identifiziert.

Tabelle 4

| Beispiel | Beispiel-Nr. für das Endprodukt der Formel I | $R^1$ | $R^2$ | Physikalische Daten |
|---|---|---|---|---|
| 62 | 28, 29 | 2-Chlorphenyl | 2-Chlorphenyl | Smp. 231°C |
| 63 | 30 | 2-Chlorphenyl | 2-Chlor-6-fluorphenyl | Smp. 223-224°C |

Die diesbezüglichen Amidrazone der Formel XII sind 2-Chlor-N'-(o-chlorphenyl)-benzamidrazon, Smp. 139-140,5°C, resp. 2-Chlor-6-fluor-N'-(o-chlorphenyl)-benzamidrazon, Smp. 162-163°C.

IV. Formulierungsbeispiele:

Beispiel 64

Ein emulgierbares Konzentrat hat folgende Zusammensetzung:

| | g/Liter |
|---|---|
| Verbindung der Formel I (Wirkstoff) | 250 |
| N-Methyl-2-pyrrolidon (Lösungsmittel) | 400 |

| | |
|---|---|
| Arkopal N100® (Nonylphenolpolyglykoläther; nichtionogenes Tensid) | 75 |
| Phenylsulfonat CAL® (Calcium-Dodecylbenzolsulfonat; anionisches Tensid) | 25 |
| Solvesso 100® (Lösungsmittel enthaltend mehr als 95 Vol.% Aromaten) | ad 1 Liter |

Der Wirkstoff und die Tenside werden in den Lösungsmitteln gelöst. Nach Verdünnen mit Wasser ergibt das so entstandene emulgierbare Konzentrat eine Emulsion, die sich gut als Spritzbrühe eignet.

## Beispiel 65

Ein Spritzpulver hat folgende Zusammensetzung:

| | | Gewichtsprozent |
|---|---|---|
| Verbindung der Formel I (Wirkstoff) | | 50 |
| Natrium-laurylsulfat (Netz-/Dispergiermittel) | | 1 |
| Natrium-lignosulfonat (Dispergiermittel) | | 2 |
| Hydratisierte Kieselsäure (ca. 87% $SiO_2$) | (inerte, pulver- | 5 |
| Kaolin (hauptsächlich $Al_2(Si_2O_5)(OH)_4$) | förmige Trägerstoffe) | 42 |
| | | 100 |

Der Wirkstoff wird mit den übrigen Formulierungskomponenten in einer geeigneten Vorrichtung homogen vermischt. Das entstandene Pulver wird nun in einem geeigneten Mahlaggregat (z.B. Stift-, Hammer-, Kugel- oder Luftstrahlmühle) auf eine für eine optimale biologische Wirksamkeit notwendige Teilchengrösse feingemahlen und hernach nochmals gemischt. Das nun vorliegende Spritzpulver wird durch Wasser spontan benetzt und ergibt gut schwebefähige, gebrauchsfertige Spritzbrühen.

## Patentansprüche

1. Verbindungen der allgemeinen Formel

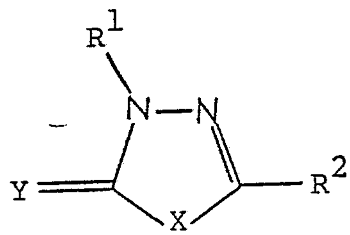

I

worin

$R^1$ gegebenenfalls mit bis zu 4 Halogenatomen, 1 bis 3 $C_{1-4}$-Alkylgruppen, 1 oder 2 Halogenmethylgruppen, einer $C_{1-3}$-Alkoxygruppe, einer $C_{1-2}$-Alkylthiogruppe, einer $C_{1-3}$-Halogenalkoxygruppe, einer $C_{1-2}$--Halogenalkylthiogruppe und/oder einer Cyanogruppe substituiertes Phenyl oder gegebenenfalls mit 1 oder 2 Halogenatomen, 1 oder 2 Methylgruppen oder einer Halogenmethylgruppe substituiertes Pyridyl,

$R^2$ mit bis zu 3 Halogenatomen, einer Methylgruppe, einer Halogenmethylgruppe und/oder 1 oder 2 Methoxygruppen substituiertes Phenyl, wobei mindestens eine der beiden o-Stellungen besetzt ist,

X Sauerstoff, Schwefel oder $NR^3$,

Y Sauerstoff oder Schwefel, und

$R^3$ Methyl, Halogenmethyl oder 2-Propinyl bedeuten.

2. Verbindungen nach Anspruch 1, worin $R^1$ eine mono- oder disubstituierte Phenylgruppe bedeutet, in der die Substituenten ein oder zwei Fluoratome, ein oder zwei Chloratome, ein Bromatom, eine oder zwei Methylgruppen, eine Trifluormethylgruppe und/oder eine Halogenmethoxygruppe sind und eine o-Stellung besetzt ist.

3. Verbindungen nach Anspruch 2, worin sich Fluor, Chlor, Brom, Methyl oder Trifluormethyl in o-Stellung befindet.

4. Verbindungen nach einem der Ansprüche 1 bis 3, worin $R^2$ eine mono- oder disubstituierte Phenylgruppe bedeutet, in der die Substituenten ein oder zwei Fluoratome, ein oder zwei Chloratome und/oder ein Bromatom sind.

5. Verbindungen nach Anspruch 4, worin $R^2$ 2-Chlorphenyl, 2-Chlor-4-fluorphenyl, 2-Chlor-6-fluorphenyl oder 2,6-Difluorphenyl bedeutet.

6. Verbindungen nach einem der Ansprüche 1 bis 5, worin X Sauerstoff bedeutet.

7. Eine Verbindung nach Anspruch 1, ausgewählt aus:

5-(2-Chlor-6-fluorphenyl)-3-(α,α,α-trifluor-o-tolyl)-1,3,4-oxadiazol-2(3H)-on,

5-(2-Chlor-6-fluorphenyl)-3-(2-chlorphenyl)-1,3,4-oxadiazol-2(3H)-on,

5-(2,6-Difluorphenyl)-3-(α,α,α-trifluor-o-tolyl)-1,3,4-oxadiazol-2(3H)-on,

5-(2-Chlor-4-fluorphenyl)-3-(2-chlorphenyl)-1,3,4-oxadiazol-2(3H)-on,

5-(2-Chlor-6-fluorphenyl)-3-(3-chlor-o-tolyl)-1,3,4-oxadiazol-2(3H)-on,

5-(2-Chlor-6-fluorphenyl)-3-(2-chlorphenyl)-1,3,4-oxadiazol-2(3H)-thion,

3-(2-Chlorphenyl)-5-(2,6-difluorphenyl)-1,3,4-oxadiazol-2(3H)-on,

3-(2-Chlorphenyl)-5-(2,6-dichlorphenyl)-1,3,4-oxadiazol-2(3H)-on,

5-(2,6-Difluorphenyl)-3-(α,α,α-trifluor-o-tolyl)-1,3,4-oxadiazol-2(3H)-thion und

3-(2-Bromphenyl)-5-(2,6-difluorphenyl)-1,3,4-oxadiazol-2(3H)-on.

8. Eine Verbindung nach Anspruch 1, ausgewählt aus 5-(2-Chlorphenyl)-3-(α,α,α-trifluor-o-tolyl)-1,3,4-

-oxadiazol-2(3H)-on und 5-(2-Chlor-6-fluorphenyl)-3-(2--fluorphenyl)-1,3,4-oxadiazol-2(3H)-on.

9. Schädlingsbekämpfungsmittel, dadurch gekennzeichnet, dass es eine wirksame Menge mindestens einer der Verbindungen der allgemeinen Formel

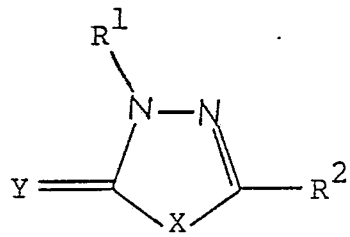

I

worin

$R^1$ gegebenenfalls mit bis zu 4 Halogenatomen, 1 bis 3 $C_{1-4}$-Alkylgruppen, 1 oder 2 Halogenmethylgruppen, einer $C_{1-3}$-Alkoxygruppe, einer $C_{1-2}$-Alkylthiogruppe, einer $C_{1-3}$-Halogenalkoxygruppe, einer $C_{1-2}$--Halogenalkylthiogruppe und/oder einer Cyanogruppe substituiertes Phenyl oder gegebenenfalls mit 1 oder 2 Halogenatomen, 1 oder 2 Methylgruppen oder einer Halogenmethylgruppe substituiertes Pyridyl,

$R^2$ mit bis zu 3 Halogenatomen, einer Methylgruppe, einer Halogenmethylgruppe und/oder 1 oder 2 Methoxygruppen substituiertes Phenyl, wobei mindestens eine der beiden o-Stellungen besetzt ist,

X Sauerstoff, Schwefel oder $NR^3$,

Y Sauerstoff oder Schwefel, und

$R^3$ Methyl, Halogenmethyl oder 2-Propinyl bedeuten,

sowie Formulierungshilfsstoffe enthält.

10. Schädlingsbekämpfungsmittel nach Anspruch 9, dadurch gekennzeichnet, dass es eine wirksame Menge mindestens einer aus der Gruppe

5-(2-Chlor-6-fluorphenyl)-3-(α,α,α-trifluor-o-tolyl)-1,3,4-oxadiazol-2(3H)-on,

5-(2-Chlor-6-fluorphenyl)-3-(2-chlorphenyl)-1,3,4-oxadiazol-2(3H)-on,

5-(2,6-Difluorphenyl)-3-(α,α,α-trifluor-o-tolyl)-1,3,4-oxadiazol-2(3H)-on,

5-(2-Chlor-4-fluorphenyl)-3-(2-chlorphenyl)-1,3,4-oxadiazol-2(3H)-on,

5-(2-Chlor-6-fluorphenyl)-3-(3-chlor-o-tolyl)-1,3,4-oxadiazol-2(3H)-on,

5-(2-Chlor-6-fluorphenyl)-3-(2-chlorphenyl)-1,3,4-oxadiazol-2(3H)-thion,

3-(2-Chlorphenyl)-5-(2,6-difluorphenyl)-1,3,4-oxadiazol-2(3H)-on,

3-(2-Chlorphenyl)-5-(2,6-dichlorphenyl)-1,3,4-oxadiazol-2(3H)-on,

5-(2,6-Difluorphenyl)-3-(α,α,α-trifluor-o-tolyl)-1,3,4-oxadiazol-2(3H)-thion und

3-(2-Bromphenyl)-5-(2,6-difluorphenyl)-1,3,4-oxadiazol-2(3H)-on

ausgewählten Verbindung sowie Formulierungshilfsstoffe enthält.

11. Schädlingsbekämpfungsmittel nach Anspruch 9, dadurch gekennzeichnet, dass es eine wirksame Menge von 5-(2-Chlorphenyl)-3-(α,α,α-trifluor-o-tolyl)-1,3,4--oxadiazol-2(3H)-on und/oder 5-(2-Chlor-6-fluorphenyl)-3--(2-fluorphenyl)-1,3,4-oxadiazol-2(3H)-on sowie Formulierungshilfsstoffe enthält.

12. Verfahren zur Herstellung von Verbindungen der allgemeinen Formel

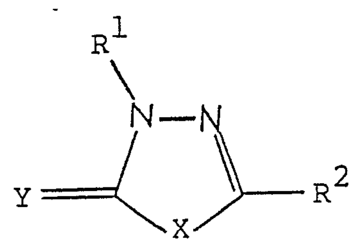

I

worin

$R^1$ gegebenenfalls mit bis zu 4 Halogenatomen, 1 bis 3 $C_{1-4}$-Alkylgruppen, 1 oder 2 Halogenmethylgruppen, einer $C_{1-3}$-Alkoxygruppe, einer $C_{1-2}$-Alkylthiogruppe, einer $C_{1-3}$-Halogenalkoxygruppe, einer $C_{1-2}$--Halogenalkylthiogruppe und/oder einer Cyanogruppe substituiertes Phenyl oder gegebenenfalls mit 1 oder 2 Halogenatomen, 1 oder 2 Methylgruppen oder einer Halogenmethylgruppe substituiertes Pyridyl,

$R^2$ mit bis zu 3 Halogenatomen, einer Methylgruppe, einer Halogenmethylgruppe und/oder 1 oder 2 Methoxygruppen substituiertes Phenyl, wobei mindestens eine der beiden o-Stellungen besetzt ist,

X Sauerstoff, Schwefel oder $NR^3$,

Y Sauerstoff oder Schwefel, und

$R^3$ Methyl, Halogenmethyl oder 2-Propinyl bedeuten,

dadurch gekennzeichnet, dass man

a) zwecks Herstellung derjenigen Verbindungen der Formel I, in denen X Sauerstoff oder Schwefel bedeutet, ein Hydrazid der allgemeinen Formel

$$R^1\text{-NH-NH-}\underset{\underset{X'}{\|}}{C}\text{-}R^2 \qquad \text{II}$$

worin $R^1$ und $R^2$ die oben angegebenen Bedeutungen besitzen und X' Sauerstoff oder Schwefel bedeutet,

mit Phosgen, Chlorameisensäure-trichlormethylester oder einem Chlorameisensäure-niederalkylester (Y= Sauerstoff) bzw. mit Thiophosgen oder Schwefelkohlenstoff (Y= Schwefel) umsetzt,

b) zwecks Herstellung derjenigen Verbindungen der Formel I, in denen X $NR^3$ und Y Sauerstoff bedeuten, ein 2,4-Dihydro-3H-1,2,4-triazol-3-on der allgemeinen Formel

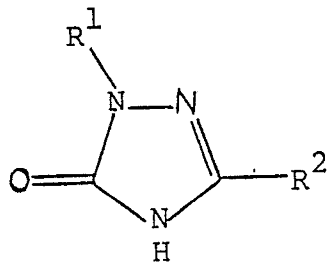

III

worin $R^1$ und $R^2$ die oben angegebenen Bedeutungen besitzen,

einer Alkylierung unterwirft, oder

c) zwecks Herstellung derjenigen Verbindungen der Formel I, in denen Y Schwefel bedeutet, eine Verbindung der allgemeinen Formel

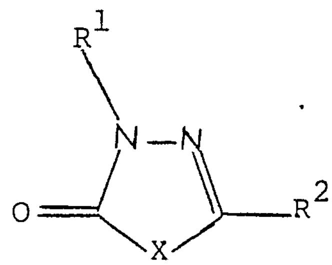

I'

worin $R^1$, $R^2$ und X die oben angegebenen Bedeutungen besitzen,

mit einem Schwefelungsmittel behandelt.

13. Verfahren zur Bekämpfung von Schädlingen, dadurch gekennzeichnet, dass man das zu schützende Gut oder die Schädlinge selbst mit einer wirksamen Menge einer Verbindung gemäss einem der Ansprüche 1 bis 8 bzw. eines Mittels gemäss einem der Ansprüche 9 bis 11 behandelt.

14. Verwendung einer Verbindung gemäss einem der Ansprüche 1 bis 8 bzw. eines Mittels gemäss einem der Ansprüche 9 bis 11 zur Bekämpfung von Schädlingen.

***

Patentansprüche für Spanien

1. Verfahren zur Herstellung von Verbindungen der allgemeinen Formel

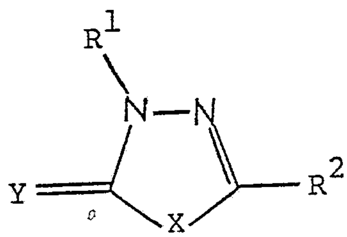

I

worin

$R^1$ gegebenenfalls mit bis zu 4 Halogenatomen, 1 bis 3 $C_{1-4}$-Alkylgruppen, 1 oder 2 Halogenmethylgruppen, einer $C_{1-3}$-Alkoxygruppe, einer $C_{1-2}$-Alkylthiogruppe, einer $C_{1-3}$-Halogenalkoxygruppe, einer $C_{1-2}$--Halogenalkylthiogruppe und/oder einer Cyanogruppe substituiertes Phenyl oder gegebenenfalls mit 1 oder 2 Halogenatomen, 1 oder 2 Methylgruppen oder einer Halogenmethylgruppe substituiertes Pyridyl,

$R^2$ mit bis zu 3 Halogenatomen, einer Methylgruppe, einer Halogenmethylgruppe und/oder 1 oder 2 Methoxygruppen substituiertes Phenyl, wobei mindestens eine der beiden o-Stellungen besetzt ist,

X Sauerstoff, Schwefel oder $NR^3$,

Y Sauerstoff oder Schwefel, und

$R^3$ Methyl, Halogenmethyl oder 2-Propinyl bedeuten,

dadurch gekennzeichnet, dass man

a) zwecks Herstellung derjenigen Verbindungen der Formel I, in denen X Sauerstoff oder Schwefel bedeutet, ein Hydrazid der allgemeinen Formel

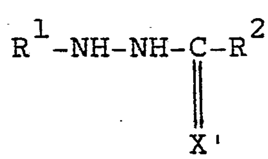

II

worin $R^1$ und $R^2$ die oben angegebenen Bedeutungen besitzen und X' Sauerstoff oder Schwefel bedeutet,

mit Phosgen, Chlorameisensäure-trichlormethylester oder einem Chlorameisensäure-niederalkylester (Y= Sauerstoff) bzw. mit Thiophosgen oder Schwefelkohlenstoff (Y= Schwefel) umsetzt,

b) zwecks Herstellung derjenigen Verbindungen der Formel I, in denen X $NR^3$ und Y Sauerstoff bedeuten, ein 2,4-Dihydro-3H-1,2,4-triazol-3-on der allgemeinen Formel

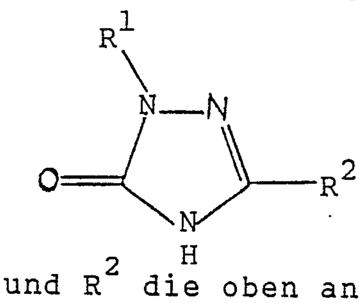

III

worin $R^1$ und $R^2$ die oben angegebenen Bedeutungen besitzen,

einer Alkylierung unterwirft, oder

c) zwecks Herstellung derjenigen Verbindungen der Formel I, in denen Y Schwefel bedeutet, eine Verbindung der allgemeinen Formel

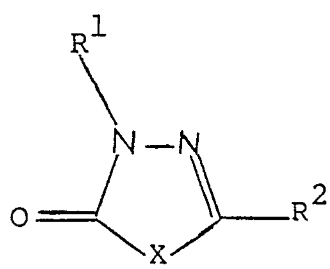

I'

worin $R^1$, $R^2$ und X die oben angegebenen Bedeutungen besitzen,

mit einem Schwefelungsmittel behandelt.

2. Verfahren nach Anspruch 1, dadurch gekennzeichnet, dass in der Variante a) das Hydrazid der Formel II mit Phosgen oder Chlorameisensäure-trichlormethylester (Y= Sauerstoff) bzw. mit Thiophosgen (Y= Schwefel) umgesetzt wird.

3. Verfahren nach Anspruch 1 oder 2, worin $R^1$ eine mono- oder disubstituierte Phenylgruppe bedeutet, in der die Substituenten ein oder zwei Fluoratome, ein oder zwei Chloratome, ein Bromatom, eine oder zwei Methylgruppen, eine Trifluormethylgruppe und/oder eine Halogenmethoxygruppe sind und eine o-Stellung besetzt ist.

4. Verfahren nach Anspruch 3, worin sich Fluor, Chlor, Brom, Methyl oder Trifluormethyl in o-Stellung befindet.

5. Verfahren nach einem der Ansprüche 1 bis 4, worin $R^2$ eine mono- oder disubstituierte Phenylgruppe bedeutet, in der die Substituenten ein oder zwei Fluoratome, ein oder zwei Chloratome und/oder ein Bromatom sind.

6. Verfahren nach Anspruch 5, worin $R^2$ 2-Chlorphenyl, 2-Chlor-4-fluorphenyl, 2-Chlor-6-fluorphenyl oder 2,6-Difluorphenyl bedeutet.

7. Verfahren nach einem der Ansprüche 1 bis 6, worin X Sauerstoff bedeutet.

8. Verfahren nach Anspruch 1 zur Herstellung einer aus der Gruppe

5-(2-Chlor-6-fluorphenyl)-3-(α,α,α-trifluor-o-tolyl)-1,3,4-oxadiazol-2(3H)-on,
5-(2-Chlor-6-fluorphenyl)-3-(2-chlorphenyl)-1,3,4-oxadiazol-2(3H)-on,
5-(2,6-Difluorphenyl)-3-(α,α,α-trifluor-o-tolyl)-1,3,4-oxadiazol-2(3H)-on,
5-(2-Chlor-4-fluorphenyl)-3-(2-chlorphenyl)-1,3,4-oxadiazol-2(3H)-on,
5-(2-Chlor-6-fluorphenyl)-3-(3-chlor-o-tolyl)-1,3,4-oxadiazol-2(3H)-on,
5-(2-Chlor-6-fluorphenyl)-3-(2-chlorphenyl)-1,3,4-oxadiazol-2(3H)-thion,

3-(2-Chlorphenyl)-5-(2,6-difluorphenyl)-1,3,4-oxadiazol-2(3H)-on,

3-(2-Chlorphenyl)-5-(2,6-dichlorphenyl)-1,3,4-oxadiazol-2(3H)-on,

5-(2,6-Difluorphenyl)-3-(α,α,α-trifluor-o-tolyl)-1,3,4-oxadiazol-2(3H)-thion und

3-(2-Bromphenyl)-5-(2,6-difluorphenyl)-1,3,4-oxadiazol-2(3H)-on

ausgewählten Verbindung.

9. Verfahren nach Anspruch 1 zur Herstellung von 5-(2-Chlorphenyl)-3-(α,α,α-trifluor-o-tolyl)-1,3,4-oxadiazol-2(3H)-on oder 5-(2-Chlor-6-fluorphenyl)-3-(2-fluorphenyl)-1,3,4-oxadiazol-2(3H)-on.

10. Schädlingsbekämpfungsmittel, dadurch gekennzeichnet, dass es eine wirksame Menge mindestens einer der Verbindungen der allgemeinen Formel

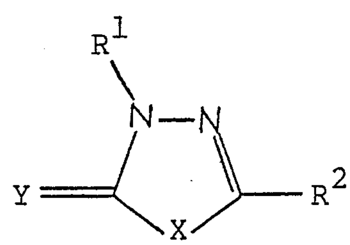

I

worin

$R^1$ gegebenenfalls mit bis zu 4 Halogenatomen, 1 bis 3 $C_{1-4}$-Alkylgruppen, 1 oder 2 Halogenmethylgruppen, einer $C_{1-3}$-Alkoxygruppe, einer $C_{1-2}$-Alkylthiogruppe, einer $C_{1-3}$-Halogenalkoxygruppe, einer $C_{1-2}$--Halogenalkylthiogruppe und/oder einer Cyanogruppe substituiertes Phenyl oder gegebenenfalls mit 1 oder 2 Halogenatomen, 1 oder 2 Methylgruppen oder einer Halogenmethylgruppe substituiertes Pyridyl,

$R^2$ mit bis zu 3 Halogenatomen, einer Methylgruppe, einer Halogenmethylgruppe und/oder 1 oder 2 Methoxygruppen substituiertes Phenyl, wobei mindestens eine der beiden o-Stellungen besetzt ist,

X Sauerstoff, Schwefel oder $NR^3$,
Y Sauerstoff oder Schwefel, und
$R^3$ Methyl, Halogenmethyl oder 2-Propinyl bedeuten,
sowie Formulierungshilfsstoffe enthält.

11. Schädlingsbekämpfungsmittel nach Anspruch 10, dadurch gekennzeichnet, dass es eine wirksame Menge mindestens einer aus der Gruppe

5-(2-Chlor-6-fluorphenyl)-3-(α,α,α-trifluor-o-tolyl)-1,3,4-oxadiazol-2(3H)-on,
5-(2-Chlor-6-fluorphenyl)-3-(2-chlorphenyl)-1,3,4-oxadiazol-2(3H)-on,
5-(2,6-Difluorphenyl)-3-(α,α,α-trifluor-o-tolyl)-1,3,4-oxadiazol-2(3H)-on,
5-(2-Chlor-4-fluorphenyl)-3-(2-chlorphenyl)-1,3,4-oxadiazol-2(3H)-on,
5-(2-Chlor-6-fluorphenyl)-3-(3-chlor-o-tolyl)-1,3,4-oxadiazol-2(3H)-on,
5-(2-Chlor-6-fluorphenyl)-3-(2-chlorphenyl)-1,3,4-oxadiazol-2(3H)-thion,
3-(2-Chlorphenyl)-5-(2,6-difluorphenyl)-1,3,4-oxadiazol-2(3H)-on,
3-(2-Chlorphenyl)-5-(2,6-dichlorphenyl)-1,3,4-oxadiazol-2(3H)-on,
5-(2,6-Difluorphenyl)-3-(α,α,α-trifluor-o-tolyl)-1,3,4-oxadiazol-2(3H)-thion und
3-(2-Bromphenyl)-5-(2,6-difluorphenyl)-1,3,4-oxadiazol-2(3H)-on

ausgewählten Verbindung sowie Formulierungshilfsstoffe enthält.

12. Schädlingsbekämpfungsmittel nach Anspruch 10, dadurch gekennzeichnet, dass es eine wirksame Menge von 5-(2-Chlorphenyl)-3-(α,α,α-trifluor-o-tolyl)-1,3,4-oxadiazol-2(3H)-on und/oder 5-(2-Chlor-6-fluorphenyl)-3-

-(2-fluorphenyl)-1,3,4-oxadiazol-2(3H)-on sowie Formulierungshilfsstoffe enthält.

13. Verfahren zur Herstellung eines Schädlingsbekämpfungsmittels gemäss Anspruch 10, dadurch gekennzeichnet, dass man mindestens eine Verbindung der allgemeinen Formel I mit Formulierungshilfsstoffen vermischt.

***